# EUROPEAN PATENT SPECIFICATION

(11) **EP 4 039 053 B1**
(45) Date of publication and mention of the grant of the patent: **04.09.2024**
(21) Application number: 20771643.2
(22) Date of filing: 08.09.2020
(51) Int. Cl.: H04W 76/15, H04L 5/00, H04W 92/20

(54) **GROUP SIGNALLING ASPECTS FOR NR-NR DUAL CONNECTIVITY (NR-DC) AND RECONFIGURATION**
GRUPPENSIGNALISIERUNGSASPEKTE FÜR DUALE NR-NR-KONNEKTIVITÄT (NR-DC) UND REKONFIGURATION
ASPECTS DE SIGNALISATION DE GROUPE POUR UNE CONNECTIVITÉ DOUBLE NR-NR (NR-DC) ET UNE RECONFIGURATION

(30) Priority: 01.10.2019 US 201962908879 P
(43) Date of publication of application: 10.08.2022
(73) Proprietor: Telefonaktiebolaget LM Ericsson (publ), 164 83 Stockholm (SE)
(72) Inventor: ARAUJO, Lian, 169 38 Solna (SE); RUGELAND, Patrik, 117 58 STOCKHOLM (SE)
(74) Representative: Ericsson
(86) International application number: PCT/SE2020/050841
(87) International publication number: WO 2021/066699

(56) References cited:
- HUAWEI ET AL: "Signalling overhead reduction for SCell Configuration", vol. RAN WG2, no. Berlin, German; 20170821 - 20170825, 20 August 2017 (2017-08-20), XP051318400, Retrieved from the Internet <URL:http://www.3gpp.org/ftp/Meetings_3GPP_SYNC/RAN2/Docs/> [retrieved on 20170820]
- ZTE CORPORATION ET AL: "Signalling overhead reduction for cell configuration and BWP configuration", vol. RAN WG2, no. Prague, Czech Republic; 20190826 - 20190830, 16 August 2019 (2019-08-16), XP051768522, Retrieved from the Internet <URL:http://www.3gpp.org/ftp/tsg_ran/WG2_RL2/TSGR2_107/Docs/R2-1910754.zip> [retrieved on 20190816]
- NOKIA ET AL: "Finalization of common SCell configuration", vol. RAN WG2, no. Busan, South Korea; 20180521 - 20180525, 20 May 2018 (2018-05-20), XP051443237, Retrieved from the Internet <URL:http://www.3gpp.org/ftp/Meetings%5F3GPP%5FSYNC/RAN2/Docs/> [retrieved on 20180520]

## Description

### TECHNICAL FIELD

This disclosure relates to group signalling aspects for NR-NR dual connectivity (NR-DC) and reconfiguration.

### BACKGROUND

Generally, all terms used herein are to be interpreted according to their ordinary meaning in the relevant technical field, unless a different meaning is clearly given and/or is implied from the context in which it is used. All references to a/an/the element, apparatus, component, means, step, etc. are to be interpreted openly as referring to at least one instance of the element, apparatus, component, means, step, etc., unless explicitly stated otherwise. The steps of any methods disclosed herein do not have to be performed in the exact order disclosed, unless a step is explicitly described as following or preceding another step and/or where it is implicit that a step must follow or precede another step. Any feature of any of the embodiments disclosed herein may be applied to any other embodiment, wherever appropriate. Likewise, any advantage of any of the embodiments may apply to any other embodiments, and vice versa. Other objectives, features and advantages of the enclosed embodiments will be apparent from the following description.

### Group signalling in LTE

Group signalling was introduced in LTE Rel-15 as part of euCA (enhancing LTE CA utilisation) Wl with the aim to reduce the required signalling to configure SCells. To achieve this, group signalling relies on the fact that many parameters, even though configured for each SCell, can actually have the same value across different SCells. Therefore, the ASN.1 signalling below (from 36.331 v 15.6.0) was introduced to enable the configuration of sCell groups, where multiple serving cells may apply the same configuration if belonging to a given SCell group.

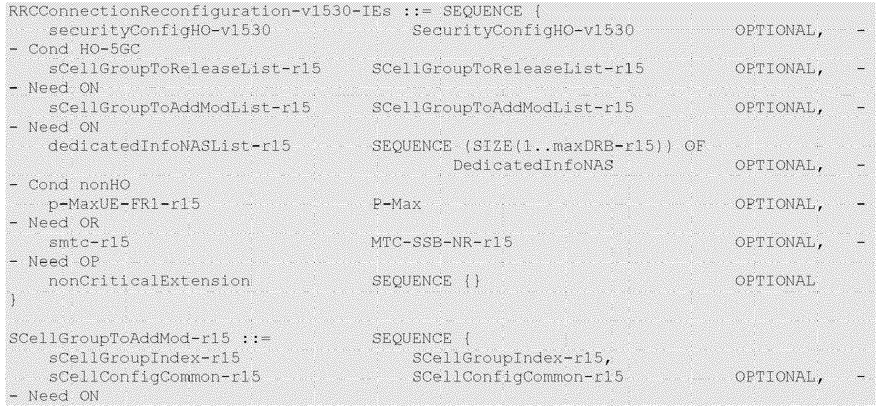

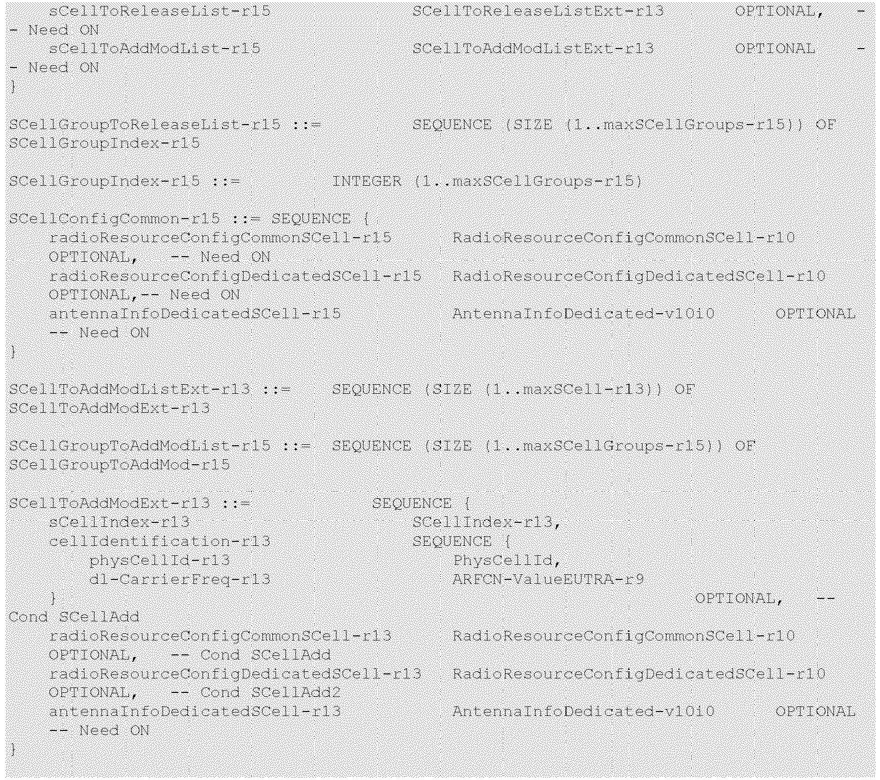

| |
|---|
| ***sCellConfigCommon*** |
| Indicates the common configuration for the SCell group. |
| ***sCellGroupIndex*** |
| Indicates the identity of SCell groups for which a common configuration is provided. |
| ***sCellGroupToAddModList, sCellGroupToAddModListSCG*** |
| Indicates the SCell group to be added or modified. E-UTRAN only configures at most 4 SCell groups per UE over all cell groups. SCell groups can only be configured for LTE SCells, and all SCells in an SCell group must belong to the same cell group. |
| ***sCellGroupToReleaseList*** |
| Indicates the SCell group to be released. |

It should be noted that since there is a field for SCG group signalling (***sCellGroupToAddModListSCG**),* this feature can be supported in the SN for both LTE-DC and NE-DC.

Concerning procedures for group signalling, the ones related to addition/modification of SCell groups are performed in 36.331 v15.6.0 as follows:

### 5.3.10.3eSCell group addition/ modification

The UE shall:
1> for each *sCellGroupIndex* value included in the *sCellGroupToAddModList* that is part of the current UE configuration (SCell group modification):
   2> for each *sCellIndex* value included in the *sCellToReleaseList* that is part of the SCell group indicated by *sCellGroupIndex* (SCell deletion from SCell group):
      3> consider the *sCellConfigCommon* of the SCell group to be not applicable for the SCell;
      3> consider the SCell not to be part of the SCell group indicated by *sCellGroupIndex*
   2> for each *sCellIndex* value included in the *sCellToAddModList* that is not part of the SCell group indicated by *sCellGroupIndex* (SCell addition to SCell group):
      3> consider the SCell to be part of the SCell group indicated by *sCellGroupIndex;*
      3> apply the SCell configuration for parameters not already configured as part of the current SCell configuration in accordance with the *sCellConfigCommon* for the SCell group;
   2> if *sCellConfigCommon* is included (modify the SCell group configuration):
      3> for each SCell that is part of the current SCell group indicated by *sCellGroupIndex:*
         4> apply the SCell configuration for parameters not already configured as part of the current SCell configuration in accordance with the *sCellConfigCommon* for the SCell group;
1> for each *sCellGroupIndex* value included in the *sCellGroupToAddModList* that is not part of the current UE configuration (SCell group addition):
   2> for each *sCellIndex* value included in the *sCellToAddModList* (SCell addition to the group):
   3> consider the SCell to be part of the SCell group indicated by *sCellGroupIndex*
   3> apply the SCell configuration for parameters not already configured as part of the current SCell configuration in accordance with the *sCellConfigCommon* for the SCell group;

### Group signalling in NR

Group signalling for NR was not yet discussed. However, from contributions submitted to RAN2 meetings (References [1], [2]), it would be similar to the approach defined for LTE (detailed above). Therefore, the present disclosure assumes that group signalling could be present in TS 38.331 in Release-16 time frame, and it would be similar to the LTE approach.

There currently exist certain challenge(s). Current group signalling is not clear on the interaction between SCell group signalling configuration and Individual SCell configuration. Namely, the following issues are present:

### Problem 1: Handling presence/absence of fields in SCell group signalling and Individual SCell configuration

It is not clear how the UE should verify "Need OR" fields (that should be released if not present) against *radioResourceConfigCommonSCell* fields configured via both Individual SCell and SCell group signalling. A "Need OR" field is optional, and if a message is received by a UE and the information element/value for the "Need OR" field is absent, the UE should discontinue/stop using/delete any existing value for the field. For instance, after the UE had an SCell configured via group signalling (*radioResourceConfigCommonSCell*), if it receives another message for delta configuration against this given SCell (i.e. a message for changing the configuration of this SCell), it is not clear whether "Need OR" fields not received in this configuration message should be released or not. The same issue will happen for NR, when NR SCell group signalling is defined, with "Need R" fields.

### Problem 2: Handling of delta signalling of SCell group configurations

Since procedures in LTE (and likely also adopted for NR) state that the UE should apply the SCell group configuration for parameters not already configured for its current SCell, current modification of an SCell group configuration cannot be performed. Even though procedures are described for this behaviour in 36.331 v15.6.0, those should only be applied for parameters not already configured for a given SCell. This approach can work when adding SCells, since any parameters included in Individual SCell configuration would take precedence over parameters included in SCell group signalling. However, once the UE applies both configurations (single and group SCell one) upon SCell addition, a subsequent change of SCell group configuration may not be effective, since the parameters therein may already be part of UEs current SCell configuration and thus will not be reconfigured according to current behaviour.

Apart from the uncertainty described above on the behaviour between SCell group signalling configuration and Individual SCell configuration, for NR-DC, it is also not clear the interaction between group signalling configuration applied to MCG and group signalling configuration applied to SCG. Therefore, a third issue is described below:

### Problem 3: Handling of SCell group configurations in NR-DC

In LTE-DC, it is possible for the MN to configure separate SCell groups for the MCG and SCG, however, for NR-DC, the MN and SN can configure an SCell group independently. This may cause a collision as both the MN and the SN may configure an SCell group with the same SCell group index comprising of different set of cells associated to the MCG or the SCG.

3GPP contribution "Signaling overhead reduction for SCell Configuration", R2-1708548, 3GPP TSG-RAN WG2 Meeting #99, Berlin, Germany, 21-25 August 2017, XP051318400, proposes mechanisms for reducing signaling overhead when SCells are configured by grouping SCells according to common characteristics.

### SUMMARY

Aspects of this disclosure provide a method performed by a wireless device, a method performed by a base station, a wireless device, a base station and a computer program product for handling group signalling of secondary cell, SCell, configuration information as defined in the claims.

### BRIEF DESCRIPTION OF THE DRAWINGS

Some of the embodiments contemplated herein will now be described more fully with reference to the accompanying drawings, in which:
Fig. 1 illustrates a wireless network in accordance with some embodiments;
Fig. 2 illustrates a User Equipment in accordance with some embodiments;
Fig. 3 shows a virtualization environment in accordance with some embodiments;
Fig. 4 shows a telecommunication network connected via an intermediate network to a host computer in accordance with some embodiments;
Fig. 5 shows a host computer communicating via a base station with a user equipment over a partially wireless connection in accordance with some embodiments;
Fig. 6 illustrates methods implemented in a communication system including a host computer, a base station and a user equipment in accordance with some embodiments;
Fig. 7 illustrates methods implemented in a communication system including a host computer, a base station and a user equipment in accordance with some embodiments;
Fig. 8 illustrates methods implemented in a communication system including a host computer, a base station and a user equipment in accordance with some embodiments;
Fig. 9 illustrates methods implemented in a communication system including a host computer, a base station and a user equipment in accordance with some embodiments;
Fig. 10 is a flow chart that illustrates one example of a method performed by a wireless device for handling signalling of SCell configuration information;
Fig. 11 is a flow chart that illustrates one example of a method performed by a base station for handling group signalling of SCell configuration information;
Fig. 12 is a flow chart that illustrates a second example of a method performed by a base station for handling group signalling of SCell configuration information;
Fig. 13 is a flow chart that illustrates a method, not covered by the claims, performed by a first network node for handling group signalling of SCell configuration information in DC;
Fig. 14 is a flow chart that illustrates a method, not covered by the claims, performed by a second network node for handling group signalling of SCell configuration information in DC;
Fig. 15 is a block diagram of a wireless device;
Fig. 16 is a block diagram of a base station;
Fig. 17 is a block diagram of a first network node, not covered by the claims; and
Fig. 18 is a block diagram of a second network node, not covered by the claims.

### DETAILED DESCRIPTION

Some of the embodiments contemplated herein will now be described more fully with reference to the accompanying drawings. Other embodiments, however, are contained within the scope of the subject matter disclosed herein, the disclosed subject matter should not be construed as limited to only the embodiments set forth herein; rather, these embodiments are provided by way of example to convey the scope of the subject matter to those skilled in the art.

It should be noted that the examples below focus on E-UTRA handling (i.e. examples are performed based on 36.331 v15.6.0), but similar examples are considered or envisaged also for the NR case (i.e. examples based on 38.331).

It should also be noted that the term "Individual SCell configurations" (or "individual configuration information") refers to configurations received in a field signalled only for a single SCell in dedicated signalling, e.g. SCellToAddModExt.

### Handling presence/absence of fields in SCell group signalling and Individual SCell configuration

### Solution 1.1: Cross-checking of included fields in an RRC message

For fields in an RRC message, where the absence implies that the corresponding field should be released, the UE should verify whether this field is not present in either a received configuration for an SCell or in a received SCell group signalling. Therefore, this approach can be used when the UE receives both Individual SCell configuration and SCell group configuration (referred to herein as "group configuration information") in the same RRC message. For instance, if the RRC message only includes an Individual SCell configuration and no SCell group configuration, the fields and conditions of the Individual SCell configuration will override any stored configuration (i.e. any field configured with SCell group which is absent in the Individual SCell configuration with "Optional Need OR" will be released).

In another example, if the RRC message only includes a SCell group configuration with some optional "Need OR" fields absent, these parameters will be released.

An example based on 36.331 v15.6.0 is given below.

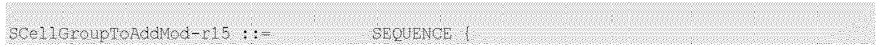

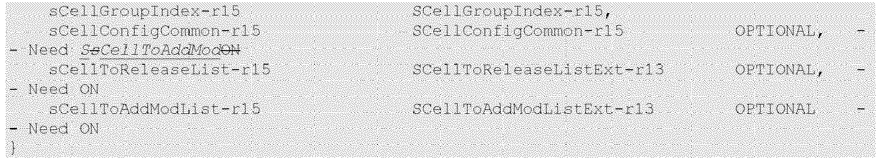

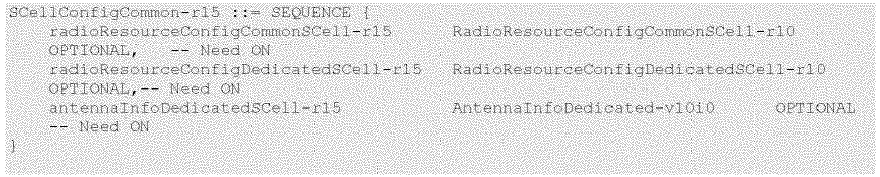

| ***RRCConnectionReconfiquration* field descriptions** |
|---|
| ***sCellConfigCommon*** |
| Indicates the common configuration for the SCell group. If this field is present. optional need OR fields within *radioResourceConfigCommonSCell* or *radioResourceConfigDedicatedSCell* should only be released if not present in neither *ScellToAddMod* nor *sCellConfigCommon.* |

| **Conditional presence** | **Explanation** |
|---|---|
| *SCellToAddMod* | The field is optionally present if *radioResourceConfigCommonSCell* or *radioResourceConfiqDedicatedSCell* are received within *SCellToAddMod.* Otherwise the field is not present. |

### Solution 1.2: UE keeps track of parameters configured via SCell group configuration or Individual SCell configuration.

In solution 1.2 the UE should remember whether a given parameter was configured via SCell group configuration or Individual SCell configuration.

In one example, parameter A and parameter B are both optional "Need OR", where parameter A is configured with an Individual SCell configuration, and parameter B is configured with a SCell group configuration.

If the UE receives an RRC message which contains only an Individual SCell configuration with parameters A and B absent, the UE will release parameter A, but maintain parameter B.

If the UE receives an RRC message which contains only a SCell group configuration with parameters A and B absent, the UE will release parameter B, but maintain parameter A.

If the UE receives an RRC message which contains only an Individual SCell configuration with parameter A absent, but including parameter B, the UE will release parameter A and replace parameter B with the received value.

If the UE receives an RRC message which contains only a SCell group configuration with parameter B absent, but including parameter A, the UE will release parameter B, but maintain the stored value of parameter A

If the UE receives an RRC message which contains both an Individual SCell configuration with parameter B and a SCell group configuration with parameter A, the UE will replace both parameters A and B with the received value (since the Individual SCell configuration had parameter A absent, this parameter should be released, but the SCell group configuration contained parameter A and thus that value is used).

An example is given below.

### - RadioResourceConfigCommon

The IE *RadioResourceConfigCommonSIB* and IE *RadioResourceConfigCommon* are used to specify common radio resource configurations in the system information and in the mobility control information, respectively, e.g., the random access parameters and the static physical layer parameters. Optional need OR fields configured in IE *RadioResourceConfisCommon* within IE *SCellToAddMod* are only released if not present in an IE *RadioResourceConfisCommon* within IE *SCellToAddMod.* Optional need OR fields configured in IE *RadioResourceConfisCommon* within IE *SCellConfigCommon* are only released if not present in an IE *RadioResourceConfisCommon* within IE *SCellConfigCommon.*

### - RadioResourceConfigDedicated

The IE *RadioResourceConfigDedicated* is used to setup/modify/release RBs, to modify the MAC main configuration, to modify the SPS configuration and to modify dedicated physical configuration. Optional need OR fields configured in IE *RadioResourceConfigDedicated* within IE *SCellToAddMod* are only released if not present in an IE *RadioResourceConfigDedicated* within IE *SCellToAddMod.* Optional need OR fields configured in IE *RadioResourceConfigDedicated* within IE *SCellConfigCommon* are only released if not present in an IE *RadioResourceConfigDedicated* within IE *SCellConfisCommon.*

### Enabling delta signalling for SCell group configuration

### Solution 2.1: Allow SCell group signalling to overwrite configurations received in previous Individual SCell configurations

In this solution, if the UE has been configured with an Individual SCell configuration for an SCell, and the UE in a subsequent RRC message receives an SCell group configuration for the SCell, any configuration received in the SCell group configuration would be overwritten for the specific SCell.

In some embodiments, any parameter with need code "Need OR" absent from the SCell group configuration which was previously configured will be released.

In other embodiments, any parameter with need code "Need OR" absent from the SCell group configuration will be ignored by the UE.

An example based on 36.331 v15.6.0 is given below.

### 5.3.10.3e SCell group addition/ modification

The UE shall:
1> for each *sCellGrouplndex* value included in the *sCellGroupToAddModList* that is part of the current UE configuration (SCell group modification):
   2> for each *sCellIndex* value included in the *sCellToReleaseList* that is part of the SCell group indicated by *sCellGroupIndex* (SCell deletion from SCell group):
      3> consider the *sCellConfigCommon* of the SCell group to be not applicable for the SCell;
      3> consider the SCell not to be part of the SCell group indicated by *sCellGroupIndex*
   2> for each *sCellIndex* value included in the *sCellToAddModList* that is not part of the SCell group indicated by *sCellGroupIndex* (SCell addition to SCell group):
      3> consider the SCell to be part of the SCell group indicated by *sCellGroupIndex;*
      3> apply the SCell configuration in accordance with the *sCellConfigCommon* for the SCell group;
   2> if *sCellConfigCommon* is included (modify the SCell group configuration):
      3> for each SCell that is part of the current SCell group indicated by *sCellGroupIndex*:
      4> apply the SCell configuration in accordance with the *sCellConfigCommon* for the SCell group;
1> for each *sCellGroupIndex* value included in the *sCellGroupToAddModList* that is not part of the current UE configuration (SCell group addition):
   2> for each *sCellIndex* value included in the *sCellToAddModList* (SCell addition to the group):
   3> consider the SCell to be part of the SCell group indicated by *sCellGroupIndex*
   3> apply the SCell configuration in accordance with the *sCellConfigCommon* for the SCell group;

In other embodiments, the signalling can be extended to include a new indication which indicates whether the SCell group configurations (including behaviour for 'need' codes) will overwrite any stored configuration. If the indication is absent, the UE will only apply configurations for parameters previously configured with SCell group configurations.

### Solution 2.2: Limit SCell group signalling for SCell addition case

In this solution the configuration of SCell group would only be provided for SCell addition case. Even though this may prevent gains from SCell group signalling for delta configuration, it would be an alternative to keep the feature in a low level of complexity while still having considerable gains from it in SCell addition case. Furthermore, this solution 2.2 may be used either with solution 1.1 or 1.2.

An example based on 36.331 v15.6.0 is given below.

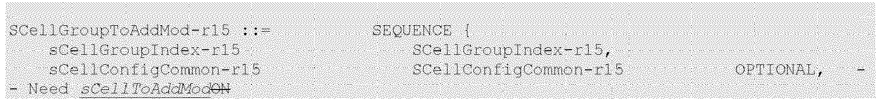

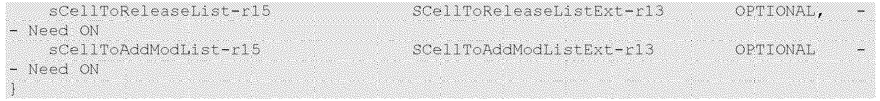

| **Conditional presence** | **Explanation** |
|---|---|
| *sCellToAddMod* | The field is optionally present upon SCell addition. Otherwise the field is not present. |

### Solution 2.3: Indicating which SCells each SCell group configuration applies to

The current definition of an SCell group includes an SCell Group Index where each SCell included is configured with the *SCellGroupIndex.*

In some embodiments, the SCellGroup configuration can instead be extended to include a list of cells for which the configurations apply to. An example configuration of this can be seen below:

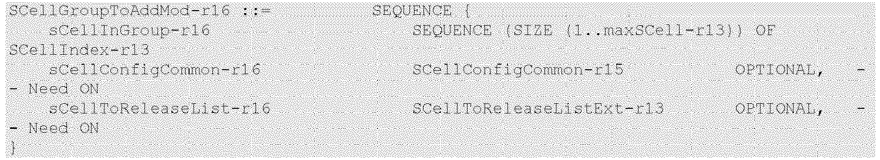

### Amendment to field conditions in SCell group configuration

In another solution that can address both Problem 1 and 2 described above, field conditions for signalling within SCell group configuration could be made entirely optional - without any further condition. In this manner, only necessary fields are included in SCell group configuration and there would be no need to define in RRC procedures that Individual SCell configuration takes precedence over SCell group configuration. Since all fields would be optional in SCell group configuration, Individual SCell configuration should handle the configuration of all fields in an RRC message where the absence means that the according field should be released. In other words, those field conditions would be checked only against Individual SCell configuration, which thus should include all relevant fields that have such condition.

An example is provided below:

### RadioResourceConfigCommon

The IE *RadioResourceConfigCommonSIB* and IE *RadioResourceConfigCommon* are used to specify common radio resource configurations in the system information and in the mobility control information, respectively, e.g., the random access parameters and the static physical layer parameters. Fields configured in IE *RadioResourceConfisCommon* within IE *SCellConfigCommon* are optional and do not follow the field conditions described in IE *RadioResourceConfigCommon.*

### - RadioResourceConfigDedicated

The IE *RadioResourceConfigDedicated* is used to setup/modify/release RBs, to modify the MAC main configuration, to modify the SPS configuration and to modify dedicated physical configuration. Fields configured in IE *RadioResourceConfigDedicated* within IE *SCellConfigCommon* are optional and do not follow the field conditions described in IE *RadioResourceConfigDedicated*

### Enabling SCell group configuration for NR-DC

To avoid RRC configuration errors due to joint use of group signalling by MN and SN in NR-DC, the MN and SN can coordinate which SCell group indices can be used by each node, e.g. via inter-node messages, standardised separation. An example is provided below, taking 38.331 v15.6.0 as a baseline, and considering SCell group signalling for NR would adopt a similar structure as the one for LTE.

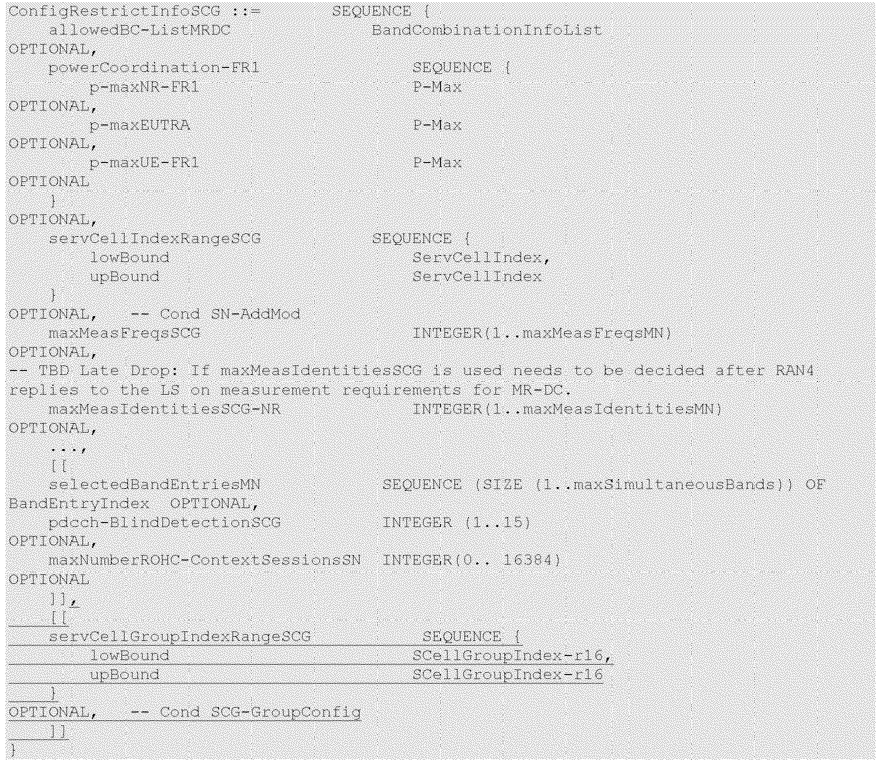

| ***CG-ConfigInfo* field descriptions** |
|---|
| ***servCellGroupIndexRangeSCG*** |
| Range of serving cell group indices that SN is allowed to configure for SCG serving cells |

| **Conditional Presence** | **Explanation** |
|---|---|
| *SCG-GroupConfig* | The field is mandatory present upon configuration of *SCellGrougToAddMod.* It is optionally present otherwise. |

Alternatively, an implicit indication can be used, where the same SCell group index is used for both the MCG and the SCG, but the UE maintains independent lists for the different cell groups. An example is provided below, as a possible description of this solution in 38.331, and considering SCell group signalling for NR would adopt a similar structure as the one for LTE.

"In NR-DC, the UE may receive two independent *SCellGroupToAddMod*:
- an *SCellGroupToAddMod,* associated with MCG, that is included in the *RRCReconfiguration* message received via SRB1; and
- an *SCellGroupToAddMod,* associated with SCG, that is included in the *RRCReconfiguration* message received via SRB3, or, alternatively, included within a *RRCReconfiguration* message embedded in a *RRCReconfiguration* message received via SRB1.

In this case, the UE maintains two independent *SCellGroupToAddMod,* and independently performs all the procedures in clause 5.3.5 for each *SCellGroupToAddMod,* unless explicitly stated otherwise.

In other embodiments, the SCell group index is not introduced, and instead the SCell group configurations include an explicit list of which SCells the configurations apply to, similar to the example in Solution 2.3.

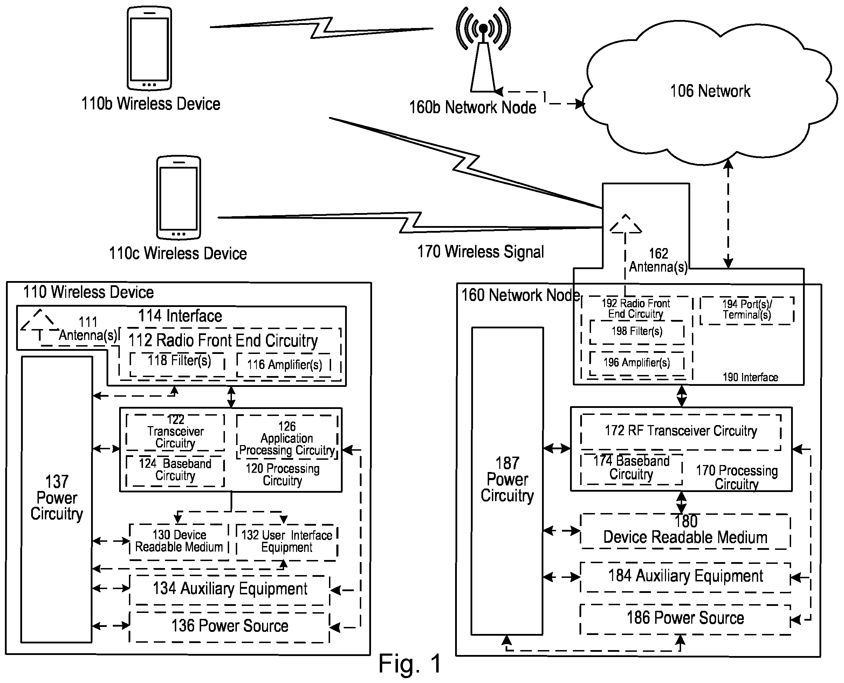

Although the subject matter described herein may be implemented in any appropriate type of system using any suitable components, the embodiments disclosed herein are described in relation to a wireless network, such as the example wireless network illustrated in Fig. 1. For simplicity, the wireless network of Fig. 1 only depicts network 106, network nodes 160 and 160b, and WDs 110, 110b, and 110c. in practice, a wireless network may further include any additional elements suitable to support communication between wireless devices or between a wireless device and another communication device, such as a landline telephone, a service provider, or any other network node or end device. Of the illustrated components, network node 160 and wireless device (WD) 110 are depicted with additional detail. The wireless network may provide communication and other types of services to one or more wireless devices to facilitate the wireless devices' access to and/or use of the services provided by, or via, the wireless network.

The wireless network may comprise and/or interface with any type of communication, telecommunication, data, cellular, and/or radio network or other similar type of system. In some embodiments, the wireless network may be configured to operate according to specific standards or other types of predefined rules or procedures. Thus, particular embodiments of the wireless network may implement communication standards, such as Global System for Mobile Communications (GSM), Universal Mobile Telecommunications System (UMTS), Long Term Evolution (LTE), and/or other suitable 2G, 3G, 4G, or 5G standards; wireless local area network (WLAN) standards, such as the IEEE 802.11 standards; and/or any other appropriate wireless communication standard, such as the Worldwide Interoperability for Microwave Access (WiMax), Bluetooth, Z-Wave and/or ZigBee standards.

Network 106 may comprise one or more backhaul networks, core networks, IP networks, public switched telephone networks (PSTNs), packet data networks, optical networks, wide-area networks (WANs), local area networks (LANs), wireless local area networks (WLANs), wired networks, wireless networks, metropolitan area networks, and other networks to enable communication between devices.

Network node 160 and WD 110 comprise various components described in more detail below. These components work together in order to provide network node and/or wireless device functionality, such as providing wireless connections in a wireless network. In different embodiments, the wireless network may comprise any number of wired or wireless networks, network nodes, base stations, controllers, wireless devices, relay stations, and/or any other components or systems that may facilitate or participate in the communication of data and/or signals whether via wired or wireless connections.

As used herein, network node refers to equipment capable, configured, arranged and/or operable to communicate directly or indirectly with a wireless device and/or with other network nodes or equipment in the wireless network to enable and/or provide wireless access to the wireless device and/or to perform other functions (e.g., administration) in the wireless network. Examples of network nodes include, but are not limited to, access points (APs) (e.g., radio access points), base stations (BSs) (e.g., radio base stations, Node Bs, evolved Node Bs (eNBs) and NR NodeBs (gNBs)). Base stations may be categorized based on the amount of coverage they provide (or, stated differently, their transmit power level) and may then also be referred to as femto base stations, pico base stations, micro base stations, or macro base stations. A base station may be a relay node or a relay donor node controlling a relay. A network node may also include one or more (or all) parts of a distributed radio base station such as centralized digital units and/or remote radio units (RRUs), sometimes referred to as Remote Radio Heads (RRHs). Such remote radio units may or may not be integrated with an antenna as an antenna integrated radio. Parts of a distributed radio base station may also be referred to as nodes in a distributed antenna system (DAS). Yet further examples of network nodes include multi-standard radio (MSR) equipment such as MSR BSs, network controllers such as radio network controllers (RNCs) or base station controllers (BSCs), base transceiver stations (BTSs), transmission points, transmission nodes, multi-cell/multicast coordination entities (MCEs), core network nodes (e.g., MSCs, MMEs), O&M nodes, OSS nodes, SON nodes, positioning nodes (e.g., E-SMLCs), and/or MDTs. As another example, a network node may be a virtual network node as described in more detail below. More generally, however, network nodes may represent any suitable device (or group of devices) capable, configured, arranged, and/or operable to enable and/or provide a wireless device with access to the wireless network or to provide some service to a wireless device that has accessed the wireless network.

In Fig. 1, network node 160 includes processing circuitry 170, device readable medium 180, interface 190, auxiliary equipment 184, power source 186, power circuitry 187, and antenna 162. Although network node 160 illustrated in the example wireless network of Fig. 1 may represent a device that includes the illustrated combination of hardware components, other embodiments may comprise network nodes with different combinations of components. It is to be understood that a network node comprises any suitable combination of hardware and/or software needed to perform the tasks, features, functions and methods disclosed herein. Moreover, while the components of network node 160 are depicted as single boxes located within a larger box, or nested within multiple boxes, in practice, a network node may comprise multiple different physical components that make up a single illustrated component (e.g., device readable medium 180 may comprise multiple separate hard drives as well as multiple RAM modules).

Similarly, network node 160 may be composed of multiple physically separate components (e.g., a NodeB component and a RNC component, or a BTS component and a BSC component, etc.), which may each have their own respective components. In certain scenarios in which network node 160 comprises multiple separate components (e.g., BTS and BSC components), one or more of the separate components may be shared among several network nodes. For example, a single RNC may control multiple NodeB's. In such a scenario, each unique NodeB and RNC pair, may in some instances be considered a single separate network node. In some embodiments, network node 160 may be configured to support multiple radio access technologies (RATs). In such embodiments, some components may be duplicated (e.g., separate device readable medium 180 for the different RATs) and some components may be reused (e.g., the same antenna 162 may be shared by the RATs). Network node 160 may also include multiple sets of the various illustrated components for different wireless technologies integrated into network node 160, such as, for example, GSM, WCDMA, LTE, NR, WiFi, or Bluetooth wireless technologies. These wireless technologies may be integrated into the same or different chip or set of chips and other components within network node 160.

Processing circuitry 170 is configured to perform any determining, calculating, or similar operations (e.g., certain obtaining operations) described herein as being provided by a network node. These operations performed by processing circuitry 170 may include processing information obtained by processing circuitry 170 by, for example, converting the obtained information into other information, comparing the obtained information or converted information to information stored in the network node, and/or performing one or more operations based on the obtained information or converted information, and as a result of said processing making a determination.

Processing circuitry 170 may comprise a combination of one or more of a microprocessor, controller, microcontroller, central processing unit, digital signal processor, application-specific integrated circuit, field programmable gate array, or any other suitable computing device, resource, or combination of hardware, software and/or encoded logic operable to provide, either alone or in conjunction with other network node 160 components, such as device readable medium 180, network node 160 functionality. For example, processing circuitry 170 may execute instructions stored in device readable medium 180 or in memory within processing circuitry 170. Such functionality may include providing any of the various wireless features, functions, or benefits discussed herein. In some embodiments, processing circuitry 170 may include a system on a chip (SOC).

In some embodiments, processing circuitry 170 may include one or more of radio frequency (RF) transceiver circuitry 172 and baseband processing circuitry 174. In some embodiments, radio frequency (RF) transceiver circuitry 172 and baseband processing circuitry 174 may be on separate chips (or sets of chips), boards, or units, such as radio units and digital units. In alternative embodiments, part or all of RF transceiver circuitry 172 and baseband processing circuitry 174 may be on the same chip or set of chips, boards, or units

In certain embodiments, some or all of the functionality described herein as being provided by a network node, base station, eNB or other such network device may be performed by processing circuitry 170 executing instructions stored on device readable medium 180 or memory within processing circuitry 170. In alternative embodiments, some or all of the functionality may be provided by processing circuitry 170 without executing instructions stored on a separate or discrete device readable medium, such as in a hard-wired manner. In any of those embodiments, whether executing instructions stored on a device readable storage medium or not, processing circuitry 170 can be configured to perform the described functionality. The benefits provided by such functionality are not limited to processing circuitry 170 alone or to other components of network node 160, but are enjoyed by network node 160 as a whole, and/or by end users and the wireless network generally.

Device readable medium 180 may comprise any form of volatile or non-volatile computer readable memory including, without limitation, persistent storage, solid-state memory, remotely mounted memory, magnetic media, optical media, random access memory (RAM), read-only memory (ROM), mass storage media (for example, a hard disk), removable storage media (for example, a flash drive, a Compact Disk (CD) or a Digital Video Disk (DVD)), and/or any other volatile or non-volatile, non-transitory device readable and/or computer-executable memory devices that store information, data, and/or instructions that may be used by processing circuitry 170. Device readable medium 180 may store any suitable instructions, data or information, including a computer program, software, an application including one or more of logic, rules, code, tables, etc. and/or other instructions capable of being executed by processing circuitry 170 and, utilized by network node 160. Device readable medium 180 may be used to store any calculations made by processing circuitry 170 and/or any data received via interface 190. In some embodiments, processing circuitry 170 and device readable medium 180 may be considered to be integrated.

Interface 190 is used in the wired or wireless communication of signalling and/or data between network node 160, network 106, and/or WDs 110. As illustrated, interface 190 comprises port(s)/terminal(s) 194 to send and receive data, for example to and from network 106 over a wired connection. Interface 190 also includes radio front end circuitry 192 that may be coupled to, or in certain embodiments a part of, antenna 162. Radio front end circuitry 192 comprises filters 198 and amplifiers 196. Radio front end circuitry 192 may be connected to antenna 162 and processing circuitry 170. Radio front end circuitry may be configured to condition signals communicated between antenna 162 and processing circuitry 170. Radio front end circuitry 192 may receive digital data that is to be sent out to other network nodes or WDs via a wireless connection. Radio front end circuitry 192 may convert the digital data into a radio signal having the appropriate channel and bandwidth parameters using a combination of filters 198 and/or amplifiers 196. The radio signal may then be transmitted via antenna 162. Similarly, when receiving data, antenna 162 may collect radio signals which are then converted into digital data by radio front end circuitry 192. The digital data may be passed to processing circuitry 170. In other embodiments, the interface may comprise different components and/or different combinations of components.

In certain alternative embodiments, network node 160 may not include separate radio front end circuitry 192, instead, processing circuitry 170 may comprise radio front end circuitry and may be connected to antenna 162 without separate radio front end circuitry 192. Similarly, in some embodiments, all or some of RF transceiver circuitry 172 may be considered a part of interface 190. In still other embodiments, interface 190 may include one or more ports or terminals 194, radio front end circuitry 192, and RF transceiver circuitry 172, as part of a radio unit (not shown), and interface 190 may communicate with baseband processing circuitry 174, which is part of a digital unit (not shown).

Antenna 162 may include one or more antennas, or antenna arrays, configured to send and/or receive wireless signals. Antenna 162 may be coupled to radio front end circuitry 190 and may be any type of antenna capable of transmitting and receiving data and/or signals wirelessly. In some embodiments, antenna 162 may comprise one or more omni-directional, sector or panel antennas operable to transmit/receive radio signals between, for example, 2 GHz and 66 GHz. An omni-directional antenna may be used to transmit/receive radio signals in any direction, a sector antenna may be used to transmit/receive radio signals from devices within a particular area, and a panel antenna may be a line of sight antenna used to transmit/receive radio signals in a relatively straight line. In some instances, the use of more than one antenna may be referred to as MIMO. In certain embodiments, antenna 162 may be separate from network node 160 and may be connectable to network node 160 through an interface or port.

Antenna 162, interface 190, and/or processing circuitry 170 may be configured to perform any receiving operations and/or certain obtaining operations described herein as being performed by a network node. Any information, data and/or signals may be received from a wireless device, another network node and/or any other network equipment. Similarly, antenna 162, interface 190, and/or processing circuitry 170 may be configured to perform any transmitting operations described herein as being performed by a network node. Any information, data and/or signals may be transmitted to a wireless device, another network node and/or any other network equipment.

Power circuitry 187 may comprise, or be coupled to, power management circuitry and is configured to supply the components of network node 160 with power for performing the functionality described herein. Power circuitry 187 may receive power from power source 186. Power source 186 and/or power circuitry 187 may be configured to provide power to the various components of network node 160 in a form suitable for the respective components (e.g., at a voltage and current level needed for each respective component). Power source 186 may either be included in, or external to, power circuitry 187 and/or network node 160. For example, network node 160 may be connectable to an external power source (e.g., an electricity outlet) via an input circuitry or interface such as an electrical cable, whereby the external power source supplies power to power circuitry 187. As a further example, power source 186 may comprise a source of power in the form of a battery or battery pack which is connected to, or integrated in, power circuitry 187. The battery may provide backup power should the external power source fail. Other types of power sources, such as photovoltaic devices, may also be used.

Alternative embodiments of network node 160 may include additional components beyond those shown in Fig. 1 that may be responsible for providing certain aspects of the network node's functionality, including any of the functionality described herein and/or any functionality necessary to support the subject matter described herein. For example, network node 160 may include user interface equipment to allow input of information into network node 160 and to allow output of information from network node 160. This may allow a user to perform diagnostic, maintenance, repair, and other administrative functions for network node 160.

As used herein, wireless device (WD) refers to a device capable, configured, arranged and/or operable to communicate wirelessly with network nodes and/or other wireless devices. Unless otherwise noted, the term WD may be used interchangeably herein with user equipment (UE). Communicating wirelessly may involve transmitting and/or receiving wireless signals using electromagnetic waves, radio waves, infrared waves, and/or other types of signals suitable for conveying information through air. In some embodiments, a WD may be configured to transmit and/or receive information without direct human interaction. For instance, a WD may be designed to transmit information to a network on a predetermined schedule, when triggered by an internal or external event, or in response to requests from the network. Examples of a WD include, but are not limited to, a smart phone, a mobile phone, a cell phone, a voice over IP (VoIP) phone, a wireless local loop phone, a desktop computer, a personal digital assistant (PDA), a wireless cameras, a gaming console or device, a music storage device, a playback appliance, a wearable terminal device, a wireless endpoint, a mobile station, a tablet, a laptop, a laptop-embedded equipment (LEE), a laptop-mounted equipment (LME), a smart device, a wireless customer-premise equipment (CPE), a vehicle-mounted wireless terminal device, etc.. A WD may support device-to-device (D2D) communication, for example by implementing a 3GPP standard for sidelink communication, vehicle-to-vehicle (V2V), vehicle-to-infrastructure (V2I), vehicle-to-everything (V2X) and may in this case be referred to as a D2D communication device. As yet another specific example, in an Internet of Things (IoT) scenario, a WD may represent a machine or other device that performs monitoring and/or measurements, and transmits the results of such monitoring and/or measurements to another WD and/or a network node. The WD may in this case be a machine-to-machine (M2M) device, which may in a 3GPP context be referred to as an MTC device. As one particular example, the WD may be a UE implementing the 3GPP narrow band internet of things (NB-loT) standard. Particular examples of such machines or devices are sensors, metering devices such as power meters, industrial machinery, or home or personal appliances (e.g. refrigerators, televisions, etc.) personal wearables (e.g., watches, fitness trackers, etc.). In other scenarios, a WD may represent a vehicle or other equipment that is capable of monitoring and/or reporting on its operational status or other functions associated with its operation. A WD as described above may represent the endpoint of a wireless connection, in which case the device may be referred to as a wireless terminal. Furthermore, a WD as described above may be mobile, in which case it may also be referred to as a mobile device or a mobile terminal.

As illustrated, wireless device 110 includes antenna 111, interface 114, processing circuitry 120, device readable medium 130, user interface equipment 132, auxiliary equipment 134, power source 136 and power circuitry 137. WD 110 may include multiple sets of one or more of the illustrated components for different wireless technologies supported by WD 110, such as, for example, GSM, WCDMA, LTE, NR, WiFi, WiMAX, or Bluetooth wireless technologies, just to mention a few. These wireless technologies may be integrated into the same or different chips or set of chips as other components within WD 110.

Antenna 111 may include one or more antennas or antenna arrays, configured to send and/or receive wireless signals, and is connected to interface 114. In certain alternative embodiments, antenna 111 may be separate from WD 110 and be connectable to WD 110 through an interface or port. Antenna 111, interface 114, and/or processing circuitry 120 may be configured to perform any receiving or transmitting operations described herein as being performed by a WD. Any information, data and/or signals may be received from a network node and/or another WD. In some embodiments, radio front end circuitry and/or antenna 111 may be considered an interface.

As illustrated, interface 114 comprises radio front end circuitry 112 and antenna 111. Radio front end circuitry 112 comprise one or more filters 118 and amplifiers 116. Radio front end circuitry 114 is connected to antenna 111 and processing circuitry 120, and is configured to condition signals communicated between antenna 111 and processing circuitry 120. Radio front end circuitry 112 may be coupled to or a part of antenna 111. In some embodiments, WD 110 may not include separate radio front end circuitry 112; rather, processing circuitry 120 may comprise radio front end circuitry and may be connected to antenna 111. Similarly, in some embodiments, some or all of RF transceiver circuitry 122 may be considered a part of interface 114. Radio front end circuitry 112 may receive digital data that is to be sent out to other network nodes or WDs via a wireless connection. Radio front end circuitry 112 may convert the digital data into a radio signal having the appropriate channel and bandwidth parameters using a combination of filters 118 and/or amplifiers 116. The radio signal may then be transmitted via antenna 111. Similarly, when receiving data, antenna 111 may collect radio signals which are then converted into digital data by radio front end circuitry 112. The digital data may be passed to processing circuitry 120. In other embodiments, the interface may comprise different components and/or different combinations of components.

Processing circuitry 120 may comprise a combination of one or more of a microprocessor, controller, microcontroller, central processing unit, digital signal processor, application-specific integrated circuit, field programmable gate array, or any other suitable computing device, resource, or combination of hardware, software, and/or encoded logic operable to provide, either alone or in conjunction with other WD 110 components, such as device readable medium 130, WD 110 functionality. Such functionality may include providing any of the various wireless features or benefits discussed herein. For example, processing circuitry 120 may execute instructions stored in device readable medium 130 or in memory within processing circuitry 120 to provide the functionality disclosed herein.

As illustrated, processing circuitry 120 includes one or more of RF transceiver circuitry 122, baseband processing circuitry 124, and application processing circuitry 126. In other embodiments, the processing circuitry may comprise different components and/or different combinations of components. In certain embodiments processing circuitry 120 of WD 110 may comprise a SOC. In some embodiments, RF transceiver circuitry 122, baseband processing circuitry 124, and application processing circuitry 126 may be on separate chips or sets of chips. In alternative embodiments, part or all of baseband processing circuitry 124 and application processing circuitry 126 may be combined into one chip or set of chips, and RF transceiver circuitry 122 may be on a separate chip or set of chips. In still alternative embodiments, part or all of RF transceiver circuitry 122 and baseband processing circuitry 124 may be on the same chip or set of chips, and application processing circuitry 126 may be on a separate chip or set of chips. In yet other alternative embodiments, part or all of RF transceiver circuitry 122, baseband processing circuitry 124, and application processing circuitry 126 may be combined in the same chip or set of chips. In some embodiments, RF transceiver circuitry 122 may be a part of interface 114. RF transceiver circuitry 122 may condition RF signals for processing circuitry 120.

In certain embodiments, some or all of the functionality described herein as being performed by a WD may be provided by processing circuitry 120 executing instructions stored on device readable medium 130, which in certain embodiments may be a computer-readable storage medium. In alternative embodiments, some or all of the functionality may be provided by processing circuitry 120 without executing instructions stored on a separate or discrete device readable storage medium, such as in a hard-wired manner. In any of those particular embodiments, whether executing instructions stored on a device readable storage medium or not, processing circuitry 120 can be configured to perform the described functionality. The benefits provided by such functionality are not limited to processing circuitry 120 alone or to other components of WD 110, but are enjoyed by WD 110 as a whole, and/or by end users and the wireless network generally.

Processing circuitry 120 may be configured to perform any determining, calculating, or similar operations (e.g., certain obtaining operations) described herein as being performed by a WD. These operations, as performed by processing circuitry 120, may include processing information obtained by processing circuitry 120 by, for example, converting the obtained information into other information, comparing the obtained information or converted information to information stored by WD 110, and/or performing one or more operations based on the obtained information or converted information, and as a result of said processing making a determination.

Device readable medium 130 may be operable to store a computer program, software, an application including one or more of logic, rules, code, tables, etc. and/or other instructions capable of being executed by processing circuitry 120. Device readable medium 130 may include computer memory (e.g., Random Access Memory (RAM) or Read Only Memory (ROM)), mass storage media (e.g., a hard disk), removable storage media (e.g., a Compact Disk (CD) or a Digital Video Disk (DVD)), and/or any other volatile or non-volatile, non-transitory device readable and/or computer executable memory devices that store information, data, and/or instructions that may be used by processing circuitry 120. In some embodiments, processing circuitry 120 and device readable medium 130 may be considered to be integrated.

User interface equipment 132 may provide components that allow for a human user to interact with WD 110. Such interaction may be of many forms, such as visual, audial, tactile, etc. User interface equipment 132 may be operable to produce output to the user and to allow the user to provide input to WD 110. The type of interaction may vary depending on the type of user interface equipment 132 installed in WD 110. For example, if WD 110 is a smart phone, the interaction may be via a touch screen; if WD 110 is a smart meter, the interaction may be through a screen that provides usage (e.g., the number of gallons used) or a speaker that provides an audible alert (e.g., if smoke is detected). User interface equipment 132 may include input interfaces, devices and circuits, and output interfaces, devices and circuits. User interface equipment 132 is configured to allow input of information into WD 110, and is connected to processing circuitry 120 to allow processing circuitry 120 to process the input information. User interface equipment 132 may include, for example, a microphone, a proximity or other sensor, keys/buttons, a touch display, one or more cameras, a USB port, or other input circuitry. User interface equipment 132 is also configured to allow output of information from WD 110, and to allow processing circuitry 120 to output information from WD 110. User interface equipment 132 may include, for example, a speaker, a display, vibrating circuitry, a USB port, a headphone interface, or other output circuitry. Using one or more input and output interfaces, devices, and circuits, of user interface equipment 132, WD 110 may communicate with end users and/or the wireless network, and allow them to benefit from the functionality described herein.

Auxiliary equipment 134 is operable to provide more specific functionality which may not be generally performed by WDs. This may comprise specialized sensors for doing measurements for various purposes, interfaces for additional types of communication such as wired communications etc. The inclusion and type of components of auxiliary equipment 134 may vary depending on the embodiment and/or scenario.

Power source 136 may, in some embodiments, be in the form of a battery or battery pack. Other types of power sources, such as an external power source (e.g., an electricity outlet), photovoltaic devices or power cells, may also be used. WD 110 may further comprise power circuitry 137 for delivering power from power source 136 to the various parts of WD 110 which need power from power source 136 to carry out any functionality described or indicated herein. Power circuitry 137 may in certain embodiments comprise power management circuitry. Power circuitry 137 may additionally or alternatively be operable to receive power from an external power source; in which case WD 110 may be connectable to the external power source (such as an electricity outlet) via input circuitry or an interface such as an electrical power cable. Power circuitry 137 may also in certain embodiments be operable to deliver power from an external power source to power source 136. This may be, for example, for the charging of power source 136. Power circuitry 137 may perform any formatting, converting, or other modification to the power from power source 136 to make the power suitable for the respective components of WD 110 to which power is supplied.

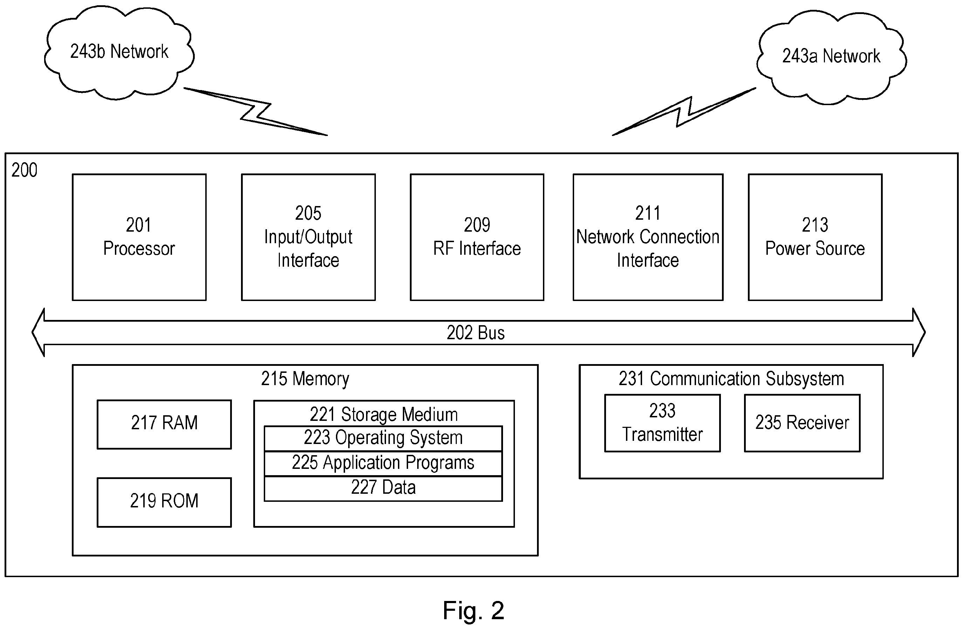

Fig. 2 illustrates one embodiment of a UE in accordance with various aspects described herein. As used herein, a user equipment or UE may not necessarily have a user in the sense of a human user who owns and/or operates the relevant device. Instead, a UE may represent a device that is intended for sale to, or operation by, a human user but which may not, or which may not initially, be associated with a specific human user (e.g., a smart sprinkler controller). Alternatively, a UE may represent a device that is not intended for sale to, or operation by, an end user but which may be associated with or operated for the benefit of a user (e.g., a smart power meter). UE 200 may be any UE identified by the 3^{rd} Generation Partnership Project (3GPP), including a NB-loT UE, a machine type communication (MTC) UE, and/or an enhanced MTC (eMTC) UE. UE 200, as illustrated in Fig. 2, is one example of a WD configured for communication in accordance with one or more communication standards promulgated by the 3^{rd} Generation Partnership Project (3GPP), such as 3GPP's GSM, UMTS, LTE, and/or 5G standards. As mentioned previously, the term WD and UE may be used interchangeable. Accordingly, although Fig. 2 is a UE, the components discussed herein are equally applicable to a WD, and vice-versa.

In Fig. 2, UE 200 includes processing circuitry 201 that is operatively coupled to input/output interface 205, radio frequency (RF) interface 209, network connection interface 211, memory 215 including random access memory (RAM) 217, read-only memory (ROM) 219, and storage medium 221 or the like, communication subsystem 231, power source 233, and/or any other component, or any combination thereof. Storage medium 221 includes operating system 223, application program 225, and data 227. In other embodiments, storage medium 221 may include other similar types of information. Certain UEs may utilize all of the components shown in Fig. 2, or only a subset of the components. The level of integration between the components may vary from one UE to another UE. Further, certain UEs may contain multiple instances of a component, such as multiple processors, memories, transceivers, transmitters, receivers, etc.

In Fig. 2, processing circuitry 201 may be configured to process computer instructions and data. Processing circuitry 201 may be configured to implement any sequential state machine operative to execute machine instructions stored as machine-readable computer programs in the memory, such as one or more hardware-implemented state machines (e.g., in discrete logic, FPGA, ASIC, etc.); programmable logic together with appropriate firmware; one or more stored program, general-purpose processors, such as a microprocessor or Digital Signal Processor (DSP), together with appropriate software; or any combination of the above. For example, the processing circuitry 201 may include two central processing units (CPUs). Data may be information in a form suitable for use by a computer.

In the depicted embodiment, input/output interface 205 may be configured to provide a communication interface to an input device, output device, or input and output device. UE 200 may be configured to use an output device via input/output interface 205. An output device may use the same type of interface port as an input device. For example, a USB port may be used to provide input to and output from UE 200. The output device may be a speaker, a sound card, a video card, a display, a monitor, a printer, an actuator, an emitter, a smartcard, another output device, or any combination thereof. UE 200 may be configured to use an input device via input/output interface 205 to allow a user to capture information into UE 200. The input device may include a touch-sensitive or presence-sensitive display, a camera (e.g., a digital camera, a digital video camera, a web camera, etc.), a microphone, a sensor, a mouse, a trackball, a directional pad, a trackpad, a scroll wheel, a smartcard, and the like. The presence-sensitive display may include a capacitive or resistive touch sensor to sense input from a user. A sensor may be, for instance, an accelerometer, a gyroscope, a tilt sensor, a force sensor, a magnetometer, an optical sensor, a proximity sensor, another like sensor, or any combination thereof. For example, the input device may be an accelerometer, a magnetometer, a digital camera, a microphone, and an optical sensor.

In Fig. 2, RF interface 209 may be configured to provide a communication interface to RF components such as a transmitter, a receiver, and an antenna. Network connection interface 211 may be configured to provide a communication interface to network 243a. Network 243a may encompass wired and/or wireless networks such as a local-area network (LAN), a wide-area network (WAN), a computer network, a wireless network, a telecommunications network, another like network or any combination thereof. For example, network 243a may comprise a Wi-Fi network. Network connection interface 211 may be configured to include a receiver and a transmitter interface used to communicate with one or more other devices over a communication network according to one or more communication protocols, such as Ethernet, TCP/IP, SONET, ATM, or the like. Network connection interface 211 may implement receiver and transmitter functionality appropriate to the communication network links (e.g., optical, electrical, and the like). The transmitter and receiver functions may share circuit components, software or firmware, or alternatively may be implemented separately.

RAM 217 may be configured to interface via bus 202 to processing circuitry 201 to provide storage or caching of data or computer instructions during the execution of software programs such as the operating system, application programs, and device drivers. ROM 219 may be configured to provide computer instructions or data to processing circuitry 201. For example, ROM 219 may be configured to store invariant low-level system code or data for basic system functions such as basic input and output (I/O), startup, or reception of keystrokes from a keyboard that are stored in a non-volatile memory. Storage medium 221 may be configured to include memory such as RAM, ROM, programmable read-only memory (PROM), erasable programmable read-only memory (EPROM), electrically erasable programmable read-only memory (EEPROM), magnetic disks, optical disks, floppy disks, hard disks, removable cartridges, or flash drives. In one example, storage medium 221 may be configured to include operating system 223, application program 225 such as a web browser application, a widget or gadget engine or another application, and data file 227. Storage medium 221 may store, for use by UE 200, any of a variety of various operating systems or combinations of operating systems.

Storage medium 221 may be configured to include a number of physical drive units, such as redundant array of independent disks (RAID), floppy disk drive, flash memory, USB flash drive, external hard disk drive, thumb drive, pen drive, key drive, high-density digital versatile disc (HD-DVD) optical disc drive, internal hard disk drive, Blu-Ray optical disc drive, holographic digital data storage (HDDS) optical disc drive, external mini-dual in-line memory module (DIMM), synchronous dynamic random access memory (SDRAM), external micro-DIMM SDRAM, smartcard memory such as a subscriber identity module or a removable user identity (SIM/RUIM) module, other memory, or any combination thereof. Storage medium 221 may allow UE 200 to access computer-executable instructions, application programs or the like, stored on transitory or non-transitory memory media, to off-load data, or to upload data. An article of manufacture, such as one utilizing a communication system may be tangibly embodied in storage medium 221, which may comprise a device readable medium.

In Fig. 2, processing circuitry 201 may be configured to communicate with network 243b using communication subsystem 231. Network 243a and network 243b may be the same network or networks or different network or networks. Communication subsystem 231 may be configured to include one or more transceivers used to communicate with network 243b. For example, communication subsystem 231 may be configured to include one or more transceivers used to communicate with one or more remote transceivers of another device capable of wireless communication such as another WD, UE, or base station of a radio access network (RAN) according to one or more communication protocols, such as IEEE 802.11, CDMA, WCDMA, GSM, LTE, UTRAN, WiMax, or the like. Each transceiver may include transmitter 233 and/or receiver 235 to implement transmitter or receiver functionality, respectively, appropriate to the RAN links (e.g., frequency allocations and the like). Further, transmitter 233 and receiver 235 of each transceiver may share circuit components, software or firmware, or alternatively may be implemented separately.

In the illustrated embodiment, the communication functions of communication subsystem 231 may include data communication, voice communication, multimedia communication, short-range communications such as Bluetooth, near-field communication, location-based communication such as the use of the global positioning system (GPS) to determine a location, another like communication function, or any combination thereof. For example, communication subsystem 231 may include cellular communication, Wi-Fi communication, Bluetooth communication, and GPS communication. Network 243b may encompass wired and/or wireless networks such as a local-area network (LAN), a wide-area network (WAN), a computer network, a wireless network, a telecommunications network, another like network or any combination thereof. For example, network 243b may be a cellular network, a Wi-Fi network, and/or a near-field network. Power source 213 may be configured to provide alternating current (AC) or direct current (DC) power to components of UE 200.

The features, benefits and/or functions described herein may be implemented in one of the components of UE 200 or partitioned across multiple components of UE 200. Further, the features, benefits, and/or functions described herein may be implemented in any combination of hardware, software or firmware. In one example, communication subsystem 231 may be configured to include any of the components described herein. Further, processing circuitry 201 may be configured to communicate with any of such components over bus 202. In another example, any of such components may be represented by program instructions stored in memory that when executed by processing circuitry 201 perform the corresponding functions described herein. In another example, the functionality of any of such components may be partitioned between processing circuitry 201 and communication subsystem 231. In another example, the non-computationally intensive functions of any of such components may be implemented in software or firmware and the computationally intensive functions may be implemented in hardware.

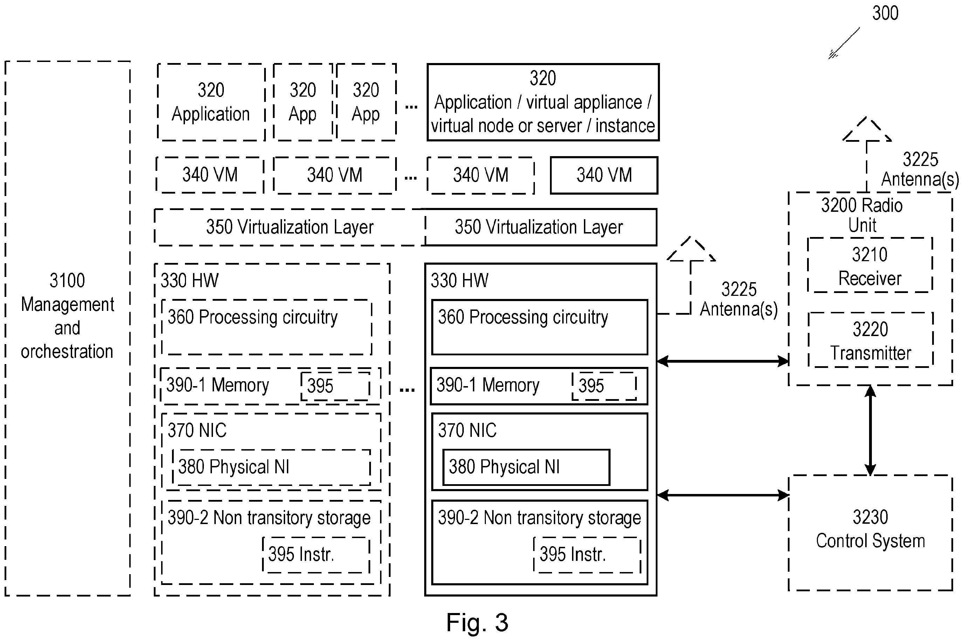

Fig. 3 is a schematic block diagram illustrating a virtualization environment 300 in which functions implemented by some embodiments may be virtualized. In the present context, virtualizing means creating virtual versions of apparatuses or devices which may include virtualizing hardware platforms, storage devices and networking resources. As used herein, virtualization can be applied to a node (e.g., a virtualized base station or a virtualized radio access node) or to a device (e.g., a UE, a wireless device or any other type of communication device) or components thereof and relates to an implementation in which at least a portion of the functionality is implemented as one or more virtual components (e.g., via one or more applications, components, functions, virtual machines or containers executing on one or more physical processing nodes in one or more networks).

In some embodiments, some or all of the functions described herein may be implemented as virtual components executed by one or more virtual machines implemented in one or more virtual environments 300 hosted by one or more of hardware nodes 330. Further, in embodiments in which the virtual node is not a radio access node or does not require radio connectivity (e.g., a core network node), then the network node may be entirely virtualized.

The functions may be implemented by one or more applications 320 (which may alternatively be called software instances, virtual appliances, network functions, virtual nodes, virtual network functions, etc.) operative to implement some of the features, functions, and/or benefits of some of the embodiments disclosed herein. Applications 320 are run in virtualization environment 300 which provides hardware 330 comprising processing circuitry 360 and memory 390. Memory 390 contains instructions 395 executable by processing circuitry 360 whereby application 320 is operative to provide one or more of the features, benefits, and/or functions disclosed herein.

Virtualization environment 300, comprises general-purpose or special-purpose network hardware devices 330 comprising a set of one or more processors or processing circuitry 360, which may be commercial off-the-shelf (COTS) processors, dedicated Application Specific Integrated Circuits (ASICs), or any other type of processing circuitry including digital or analog hardware components or special purpose processors. Each hardware device may comprise memory 390-1 which may be non-persistent memory for temporarily storing instructions 395 or software executed by processing circuitry 360. Each hardware device may comprise one or more network interface controllers (NICs) 370, also known as network interface cards, which include physical network interface 380. Each hardware device may also include non-transitory, persistent, machine-readable storage media 390-2 having stored therein software 395 and/or instructions executable by processing circuitry 360. Software 395 may include any type of software including software for instantiating one or more virtualization layers 350 (also referred to as hypervisors), software to execute virtual machines 340 as well as software allowing it to execute functions, features and/or benefits described in relation with some embodiments described herein.

Virtual machines 340, comprise virtual processing, virtual memory, virtual networking or interface and virtual storage, and may be run by a corresponding virtualization layer 350 or hypervisor. Different embodiments of the instance of virtual appliance 320 may be implemented on one or more of virtual machines 340, and the implementations may be made in different ways.

During operation, processing circuitry 360 executes software 395 to instantiate the hypervisor or virtualization layer 350, which may sometimes be referred to as a virtual machine monitor (VMM). Virtualization layer 350 may present a virtual operating platform that appears like networking hardware to virtual machine 340.

As shown in Fig. 3, hardware 330 may be a standalone network node with generic or specific components. Hardware 330 may comprise antenna 3225 and may implement some functions via virtualization. Alternatively, hardware 330 may be part of a larger cluster of hardware (e.g. such as in a data center or customer premise equipment (CPE)) where many hardware nodes work together and are managed via management and orchestration (MANO) 3100, which, among others, oversees lifecycle management of applications 320.

Virtualization of the hardware is in some contexts referred to as network function virtualization (NFV). NFV may be used to consolidate many network equipment types onto industry standard high volume server hardware, physical switches, and physical storage, which can be located in data centers, and customer premise equipment.

In the context of NFV, virtual machine 340 may be a software implementation of a physical machine that runs programs as if they were executing on a physical, non-virtualized machine. Each of virtual machines 340, and that part of hardware 330 that executes that virtual machine, be it hardware dedicated to that virtual machine and/or hardware shared by that virtual machine with others of the virtual machines 340, forms a separate virtual network elements (VNE).

Still in the context of NFV, Virtual Network Function (VNF) is responsible for handling specific network functions that run in one or more virtual machines 340 on top of hardware networking infrastructure 330 and corresponds to application 320 in Fig. 3.

In some embodiments, one or more radio units 3200 that each include one or more transmitters 3220 and one or more receivers 3210 may be coupled to one or more antennas 3225. Radio units 3200 may communicate directly with hardware nodes 330 via one or more appropriate network interfaces and may be used in combination with the virtual components to provide a virtual node with radio capabilities, such as a radio access node or a base station.

In some embodiments, some signalling can be effected with the use of control system 3230 which may alternatively be used for communication between the hardware nodes 330 and radio units 3200.

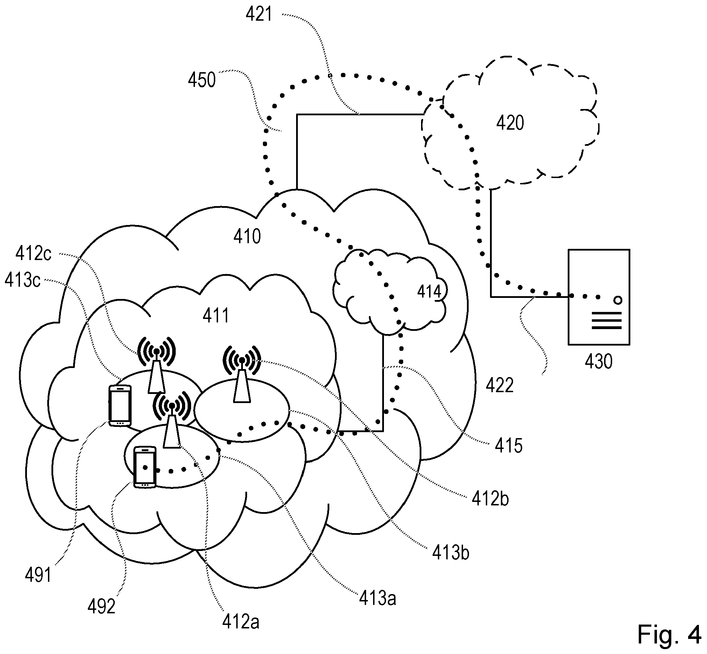

Fig. 4 shows a telecommunication network connected via an intermediate network to a host computer in accordance with some embodiments.

With reference to Fig. 4, in accordance with an embodiment, a communication system includes telecommunication network 410, such as a 3GPP-type cellular network, which comprises access network 411, such as a radio access network, and core network 414. Access network 411 comprises a plurality of base stations 412a, 412b, 412c, such as NBs, eNBs, gNBs or other types of wireless access points, each defining a corresponding coverage area 413a, 413b, 413c. Each base station 412a, 412b, 412c is connectable to core network 414 over a wired or wireless connection 415. A first UE 491 located in coverage area 413c is configured to wirelessly connect to, or be paged by, the corresponding base station 412c. A second UE 492 in coverage area 413a is wirelessly connectable to the corresponding base station 412a. While a plurality of UEs 491, 492 are illustrated in this example, the disclosed embodiments are equally applicable to a situation where a sole UE is in the coverage area or where a sole UE is connecting to the corresponding base station 412.

Telecommunication network 410 is itself connected to host computer 430, which may be embodied in the hardware and/or software of a standalone server, a cloud-implemented server, a distributed server or as processing resources in a server farm. Host computer 430 may be under the ownership or control of a service provider, or may be operated by the service provider or on behalf of the service provider. Connections 421 and 422 between telecommunication network 410 and host computer 430 may extend directly from core network 414 to host computer 430 or may go via an optional intermediate network 420. Intermediate network 420 may be one of, or a combination of more than one of, a public, private or hosted network; intermediate network 420, if any, may be a backbone network or the Internet; in particular, intermediate network 420 may comprise two or more sub-networks (not shown).

The communication system of Fig. 4 as a whole enables connectivity between the connected UEs 491, 492 and host computer 430. The connectivity may be described as an over-the-top (OTT) connection 450. Host computer 430 and the connected UEs 491, 492 are configured to communicate data and/or signalling via OTT connection 450, using access network 411, core network 414, any intermediate network 420 and possible further infrastructure (not shown) as intermediaries. OTT connection 450 may be transparent in the sense that the participating communication devices through which OTT connection 450 passes are unaware of routing of uplink and downlink communications. For example, base station 412 may not or need not be informed about the past routing of an incoming downlink communication with data originating from host computer 430 to be forwarded (e.g., handed over) to a connected UE 491. Similarly, base station 412 need not be aware of the future routing of an outgoing uplink communication originating from the UE 491 towards the host computer 430.

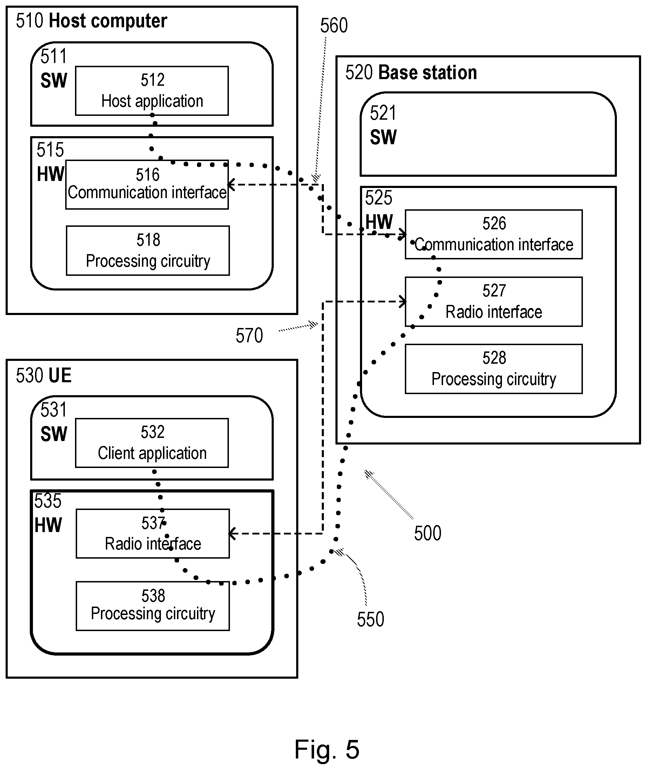

Example implementations, in accordance with an embodiment, of the UE, base station and host computer discussed in the preceding paragraphs will now be described with reference to Fig. 5. In communication system 500, host computer 510 comprises hardware 515 including communication interface 516 configured to set up and maintain a wired or wireless connection with an interface of a different communication device of communication system 500. Host computer 510 further comprises processing circuitry 518, which may have storage and/or processing capabilities. In particular, processing circuitry 518 may comprise one or more programmable processors, application-specific integrated circuits, field programmable gate arrays or combinations of these (not shown) adapted to execute instructions. Host computer 510 further comprises software 511, which is stored in or accessible by host computer 510 and executable by processing circuitry 518. Software 511 includes host application 512. Host application 512 may be operable to provide a service to a remote user, such as UE 530 connecting via OTT connection 550 terminating at UE 530 and host computer 510. In providing the service to the remote user, host application 512 may provide user data which is transmitted using OTT connection 550.

Communication system 500 further includes base station 520 provided in a telecommunication system and comprising hardware 525 enabling it to communicate with host computer 510 and with UE 530. Hardware 525 may include communication interface 526 for setting up and maintaining a wired or wireless connection with an interface of a different communication device of communication system 500, as well as radio interface 527 for setting up and maintaining at least wireless connection 570 with UE 530 located in a coverage area (not shown in Fig. 5) served by base station 520. Communication interface 526 may be configured to facilitate connection 560 to host computer 510. Connection 560 may be direct or it may pass through a core network (not shown in Fig. 5) of the telecommunication system and/or through one or more intermediate networks outside the telecommunication system. In the embodiment shown, hardware 525 of base station 520 further includes processing circuitry 528, which may comprise one or more programmable processors, application-specific integrated circuits, field programmable gate arrays or combinations of these (not shown) adapted to execute instructions. Base station 520 further has software 521 stored internally or accessible via an external connection.

Communication system 500 further includes UE 530 already referred to. Its hardware 535 may include radio interface 537 configured to set up and maintain wireless connection 570 with a base station serving a coverage area in which UE 530 is currently located. Hardware 535 of UE 530 further includes processing circuitry 538, which may comprise one or more programmable processors, application-specific integrated circuits, field programmable gate arrays or combinations of these (not shown) adapted to execute instructions. UE 530 further comprises software 531, which is stored in or accessible by UE 530 and executable by processing circuitry 538. Software 531 includes client application 532. Client application 532 may be operable to provide a service to a human or non-human user via UE 530, with the support of host computer 510. In host computer 510, an executing host application 512 may communicate with the executing client application 532 via OTT connection 550 terminating at UE 530 and host computer 510. In providing the service to the user, client application 532 may receive request data from host application 512 and provide user data in response to the request data. OTT connection 550 may transfer both the request data and the user data. Client application 532 may interact with the user to generate the user data that it provides.

It is noted that host computer 510, base station 520 and UE 530 illustrated in Fig. 5 may be similar or identical to host computer 430, one of base stations 412a, 412b, 412c and one of UEs 491, 492 of Fig. 4, respectively. This is to say, the inner workings of these entities may be as shown in Fig. 5 and independently, the surrounding network topology may be that of Fig. 4.

In Fig. 5, OTT connection 550 has been drawn abstractly to illustrate the communication between host computer 510 and UE 530 via base station 520, without explicit reference to any intermediary devices and the precise routing of messages via these devices. Network infrastructure may determine the routing, which it may be configured to hide from UE 530 or from the service provider operating host computer 510, or both. While OTT connection 550 is active, the network infrastructure may further take decisions by which it dynamically changes the routing (e.g., on the basis of load balancing consideration or reconfiguration of the network).

Wireless connection 570 between UE 530 and base station 520 is in accordance with the teachings of the embodiments described throughout this disclosure. One or more of the various embodiments improve the performance of OTT services provided to UE 530 using OTT connection 550, in which wireless connection 570 forms the last segment. More precisely, the teachings of these embodiments enable delta signalling of SCell group configurations which reduces the signalling load and/or reduce the complexity of SCell group configuration where delta signalling is not enabled, and thereby provide benefits such as reduced user waiting time and improved SCell group configuration.

A measurement procedure may be provided for the purpose of monitoring data rate, latency and other factors on which the one or more embodiments improve. There may further be an optional network functionality for reconfiguring OTT connection 550 between host computer 510 and UE 530, in response to variations in the measurement results. The measurement procedure and/or the network functionality for reconfiguring OTT connection 550 may be implemented in software 511 and hardware 515 of host computer 510 or in software 531 and hardware 535 of UE 530, or both. In embodiments, sensors (not shown) may be deployed in or in association with communication devices through which OTT connection 550 passes; the sensors may participate in the measurement procedure by supplying values of the monitored quantities exemplified above, or supplying values of other physical quantities from which software 511, 531 may compute or estimate the monitored quantities. The reconfiguring of OTT connection 550 may include message format, retransmission settings, preferred routing etc.; the reconfiguring need not affect base station 520, and it may be unknown or imperceptible to base station 520. Such procedures and functionalities may be known and practiced in the art. In certain embodiments, measurements may involve proprietary UE signalling facilitating host computer 510's measurements of throughput, propagation times, latency and the like. The measurements may be implemented in that software 511 and 531 causes messages to be transmitted, in particular empty or 'dummy' messages, using OTT connection 550 while it monitors propagation times, errors etc.

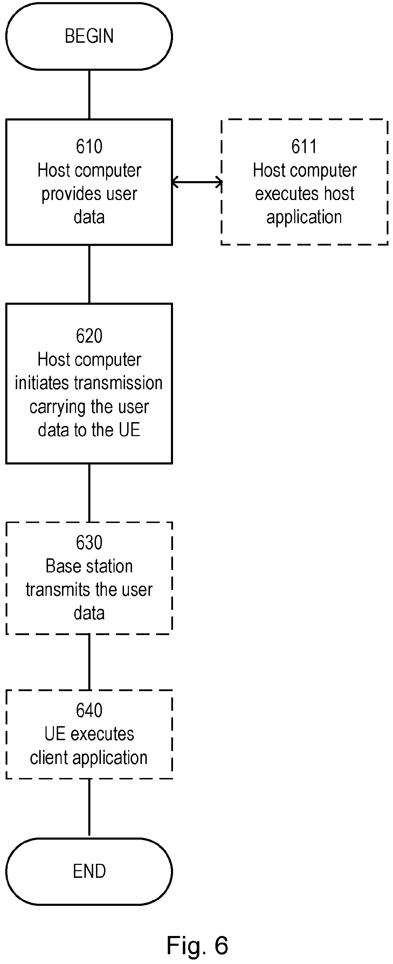

Fig. 6 is a flowchart illustrating a method implemented in a communication system, in accordance with one embodiment. The communication system includes a host computer, a base station and a UE which may be those described with reference to Figs. 4 and 5. For simplicity of the present disclosure, only drawing references to Fig. 6 will be included in this section. In step 610, the host computer provides user data. In substep 611 (which may be optional) of step 610, the host computer provides the user data by executing a host application. In step 620, the host computer initiates a transmission carrying the user data to the UE. In step 630 (which may be optional), the base station transmits to the UE the user data which was carried in the transmission that the host computer initiated, in accordance with the teachings of the embodiments described throughout this disclosure. In step 640 (which may also be optional), the UE executes a client application associated with the host application executed by the host computer.

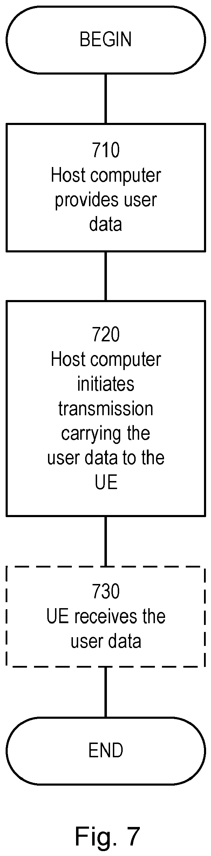

Fig. 7 is a flowchart illustrating a method implemented in a communication system, in accordance with one embodiment. The communication system includes a host computer, a base station and a UE which may be those described with reference to Figs. 4 and 5. For simplicity of the present disclosure, only drawing references to Fig. 7 will be included in this section. In step 710 of the method, the host computer provides user data. In an optional substep (not shown) the host computer provides the user data by executing a host application. In step 720, the host computer initiates a transmission carrying the user data to the UE. The transmission may pass via the base station, in accordance with the teachings of the embodiments described throughout this disclosure. In step 730 (which may be optional), the UE receives the user data carried in the transmission.

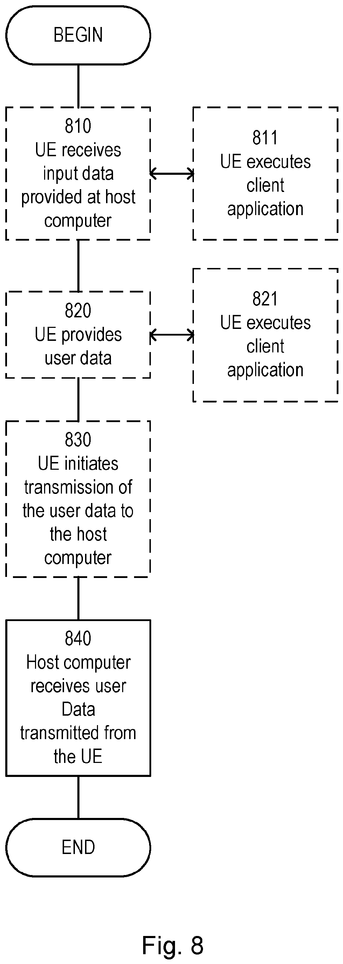

Fig. 8 is a flowchart illustrating a method implemented in a communication system, in accordance with one embodiment. The communication system includes a host computer, a base station and a UE which may be those described with reference to Figs. 4 and 5. For simplicity of the present disclosure, only drawing references to Fig. 8 will be included in this section. In step 810 (which may be optional), the UE receives input data provided by the host computer. Additionally or alternatively, in step 820, the UE provides user data. In substep 821 (which may be optional) of step 820, the UE provides the user data by executing a client application. In substep 811 (which may be optional) of step 810, the UE executes a client application which provides the user data in reaction to the received input data provided by the host computer. In providing the user data, the executed client application may further consider user input received from the user. Regardless of the specific manner in which the user data was provided, the UE initiates, in substep 830 (which may be optional), transmission of the user data to the host computer. In step 840 of the method, the host computer receives the user data transmitted from the UE, in accordance with the teachings of the embodiments described throughout this disclosure.

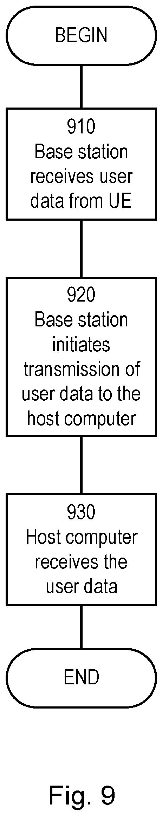

Fig. 9 is a flowchart illustrating a method implemented in a communication system, in accordance with one embodiment. The communication system includes a host computer, a base station and a UE which may be those described with reference to Figs. 4 and 5. For simplicity of the present disclosure, only drawing references to Fig. 9 will be included in this section. In step 910 (which may be optional), in accordance with the teachings of the embodiments described throughout this disclosure, the base station receives user data from the UE. In step 920 (which may be optional), the base station initiates transmission of the received user data to the host computer. In step 930 (which may be optional), the host computer receives the user data carried in the transmission initiated by the base station.

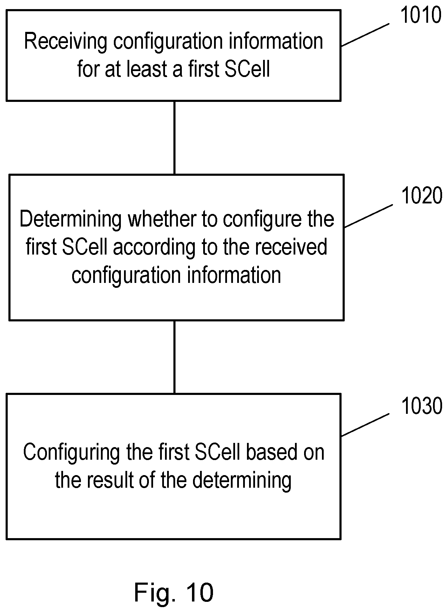

Fig. 10 is a flowchart illustrating a method performed by a wireless device for handling group signalling of SCell configuration information. The method begins at step 1010 with receiving configuration information for at least a first SCell. The configuration information comprises values for one or more fields in a set of fields. In some embodiments, the configuration information is received in a RRC message. The received configuration information is group configuration information comprising values for the one or more fields that is applicable to a group of SCells that includes the first SCell.

The method shown in Fig. 10 further comprises a step 1020 of determining whether to configure the first SCell according to the received configuration information. At step 1030, the method comprises configuring the first SCell based on the result of the step of determining.

In some embodiments, the method depicted in Fig. 10 further comprises a step of utilising radio resources from the configured first SCell. The method may also comprise steps of providing user data and forwarding the user data to a host computer via the first SCell.

In some embodiments, the set of fields comprises a first field, and the first field is a field for which an absence of a value in the received configuration information is to cause a default action in the wireless device to release, discard, delete or stop using a previously-stored value for the first field. In some such embodiments, the received configuration information does not include a value for the first field.

In some of these embodiments, in line with solution 1.1 above, the received configuration information is group configuration information and step 1020 comprises determining whether the wireless device has a value for the first field for the first SCell obtained from individual configuration information. The individual configuration information comprises values for the one or more fields that is applicable to the first SCell. Step 1020 also comprises determining whether to perform the default action for the first field based on whether the wireless device has a value for the first field for the first SCell obtained from individual configuration information.

Determining whether to perform the default action may comprise determining that the default action is to be performed if, or only if, the wireless device does not have a value for the first field for the first SCell obtained from the individual configuration information. Otherwise, it is determined that the value for the first field for the first SCell obtained from the individual configuration information is to be maintained.

In some embodiments, step 1010 further comprises receiving the individual configuration information. In alternative embodiments, the individual configuration information is received prior to receiving the group configuration information.

In some embodiments, in line with solutions 1.1 and 2.1 above, step 1020 comprises determining that the values for the one or more fields in the received configuration information are to be used for the first SCell in place of any previously-stored values for the one or more fields. In some such embodiments, in accordance with solution 2.1, the received configuration information does not include a value for the first field, and step 1020 further comprises either: performing the default action for the first field and releasing, discarding, deleting or stopping using the previously-stored value for the first field; or ignoring the absent value for the first field and maintaining the previously-stored value for the first field for the first SCell.

Optionally, the method of Fig. 10 can further comprise receiving an indication relating to the received configuration information. This indication indicates whether the received configuration information is to be used to replace previously-stored values for the one or more fields. In some such embodiments, the received configuration information is group configuration information. If in these embodiments the indication is absent, or if the indication indicates that the received group configuration information is not to replace previously-stored values for the one or more fields from individual configuration information, then step 1020 comprises determining to use a value for a field in the set of fields in the group configuration information if, or only if, the previously-stored value of the field in the set of fields was received in group configuration information.

In alternative embodiments, in line with solution 1.2 above, the wireless device has a stored value for the first field that was previously received in individual configuration information or group configuration information. The individual configuration information comprised values for the one or more fields that are applicable to the first SCell, and the group configuration information comprised values for the one or more fields that are applicable to a group of SCells including the first SCell. In such embodiments, step 1010 comprises receiving one of individual configuration information and group configuration information. The determining in step 1020 is based on whether the stored value for the first field was previously received in individual configuration information or group configuration information and whether the received configuration information is individual configuration information or group configuration information. In some of these embodiments, if the stored value of the first field was previously received in individual configuration information and the received configuration information is individual configuration information, it is determined that the default action for the first field is to be performed to release, discard, delete or stop using the previously-stored value for the first field. If, on the other hand, the stored value of the first field was previously received in individual configuration information and the received configuration information is group configuration information, it is determined that the previously-stored value for the first field is to be used. Finally, if the stored value of the first field was previously received in group configuration information and the received configuration information is individual configuration information, it is determined that the previously-stored value for the first field is to be used.

In accordance with solution 2.2 above, in some embodiments the received configuration information is for adding the first SCell as a SCell for the wireless device. The received configuration information may be only for adding the first SCell as a SCell for the wireless device. In these embodiments, the received configuration information does not relate to a SCell that is an existing SCell for the wireless device.

In some embodiments, in line with solution 2.3 above, the group configuration information comprises values for the one or more fields that are applicable to a group of SCells including the first SCell, and the group configuration information further comprises a list of SCells in the group of SCells that the group configuration information applies to. In alternative embodiments, the group configuration information comprises values for the one or more fields that are applicable to a group of SCells, the group configuration information further comprises a first list of SCells in a MCG that the group configuration information applies to, and a second list of SCells in a SCG that the group configuration information applies to. In such embodiments, the first list and the second list may be identified by a same index or by different indices.

In some embodiments, the received group configuration information comprises values for the one or more fields that are applicable to a group of SCells including the first SCell. Each of the fields is an optional field.

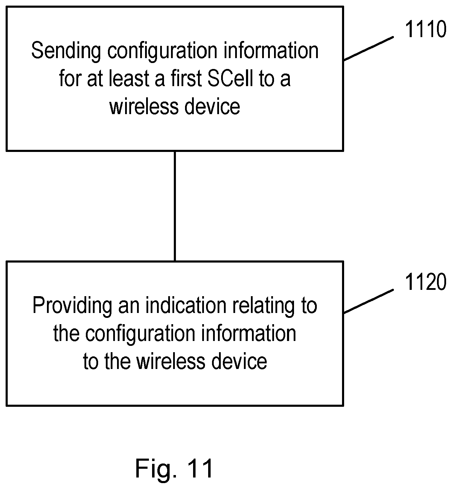

Fig. 11 is a flowchart illustrating a method performed by a base station for handling group signalling of SCell configuration information. The method begins at step 1110 with sending configuration information for at least a first SCell to a wireless device. The configuration information comprises values for one or more fields in a set of fields. The method further comprises step 1120 of providing an indication relating to the configuration information to the wireless device. The indication indicates whether the configuration information is to be used to replace previously-stored values for the one or more fields.

Optionally, the method further comprises determining whether the values for the one or more fields in the group configuration information are to replace previously-stored values for the one or more fields from individual configuration information.

In some embodiments, an indication that values for the one or more fields in the group configuration information are not to replace previously-stored values for the one or more fields from individual configuration information is provided by a negative indication, or by an absence of a positive indication.

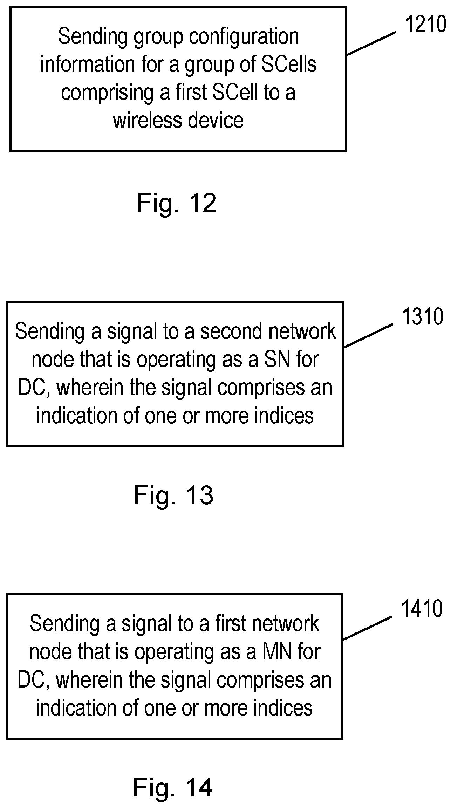

Fig. 12 is a flowchart illustrating a method performed by a base station for handling group signalling of SCell configuration information in accordance with solution 2.3 above. The method comprises step 1210 of sending group configuration information for a group of SCells comprising a first SCell to a wireless device. The group configuration information comprises values for one or more fields in a set of fields. In some embodiments, the group configuration information further comprises a list of SCells in the group of SCells that the group configuration information applies to. Alternatively, the group configuration information may comprise values for one or more fields in a set of fields, where each of the fields is an optional field.

In some embodiments, the method further comprises obtaining user data and forwarding the user data to a host computer or a wireless device via the first SCell.

Figs. 13 and 14 relate to the solutions to problem 3 described above.

Fig. 13 is a flowchart illustrating a method performed by a first network node for handling group signalling of SCell configuration information in DC. This method is not covered by the claims and is included for illustration purpose. The first network node is operating as a MN for DC. The method comprises a step 1310 of sending a signal to a second network node that is operating as a SN for DC. The signal comprises an indication of one or more indices that can be used by the MN and/or the SN for identifying a group of SCells that can be configured using group configuration information. The group configuration information comprises values for one or more fields that are applicable to a group of SCells.

Fig. 14 is a flowchart illustrating a method performed by a second network node for handling group signalling of SCell configuration information in DC. This method is not covered by the claims and is included for illustration purpose. The second network node is operating as a SN for DC. The method comprises a step 1410 of sending a signal to a first network node that is operating as a MN for DC. The signal comprises an indication of one or more indices that can be used by the MN and/or the SN for identifying a group of SCells that can be configured using group configuration information. The group configuration information comprises values for one or more fields that are applicable to a group of SCells. In some embodiments, DC is New Radio-DC.

The signal in Fig. 13 or 14 can be an explicit indication or an implicit indication. In some embodiments, the indication indicates a range of indices that can be used by the MN and/or the SN. In some such embodiments, the indication indicates a respective range of indices that can be used by the MN and the SN. In some embodiments, the indication indicates an index to be used for both a MCG managed by the MN, and a SCG managed by the SN.

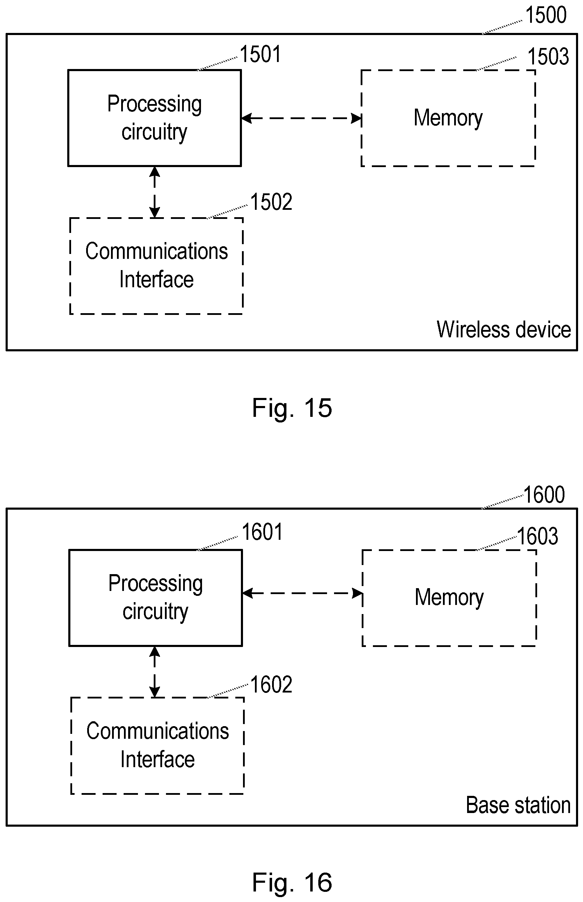

Fig. 15 is a block diagram of a wireless device 1500 according to various embodiments. The wireless device 1500 comprises processing circuitry (or logic) 1501. The processing circuitry 1501 controls the operation of the wireless device 1500 and can implement the methods described herein in relation to a wireless device 1500. The processing circuitry 1501 can comprise one or more processors, processing units, multi-core processors or modules that are configured or programmed to control the wireless device 1500 in the manner described herein. In particular implementations, the processing circuitry 1501 can comprise a plurality of software and/or hardware modules that are each configured to perform, or are for performing, individual or multiple steps of the methods described herein in relation to wireless devices.

In some embodiments, the wireless device 1500 may optionally comprise a communications interface 1502. The communications interface 1502 can be for use in communicating with other nodes, for example a base station or other network node.

For example, the communications interface 1502 can be configured to transmit to and/or receive from other nodes requests, resources, information, data, signals, or similar. The processing circuitry 1501 may be configured to control the communications interface 1502 to transmit to and/or receive from other nodes requests, resources, information, data, signals, or similar.

Optionally, the wireless device 1500 may comprise a memory 1503. In some embodiments, the memory 1503 can be configured to store program code that can be executed by the processing circuitry 1501 to perform the methods described herein in relation to wireless devices. Alternatively or in addition, the memory 1503 can be configured to store any requests, resources, information, data, signals, or similar that are described herein. The processing circuitry 1501 may be configured to control the memory 1503 to store any requests, resources, information, data, signals, or similar that are described herein.

Fig. 16 is a block diagram of a base station 1600 according to various embodiments. The base station 1600 comprises processing circuitry (or logic) 1601. The processing circuitry 1601 controls the operation of the base station 1600 and can implement the methods described herein in relation to a base station 1600. The processing circuitry 1601 can comprise one or more processors, processing units, multi-core processors or modules that are configured or programmed to control the base station 1600 in the manner described herein. In particular implementations, the processing circuitry 1601 can comprise a plurality of software and/or hardware modules that are each configured to perform, or are for performing, individual or multiple steps of the methods described herein in relation to base stations.

In some embodiments, the base station 1600 may optionally comprise a communications interface 1602. The communications interface 1602 can be for use in communicating with other nodes, for example a wireless device, another base station or other network node.

For example, the communications interface 1602 can be configured to transmit to and/or receive from other nodes requests, resources, information, data, signals, or similar. The processing circuitry 1601 may be configured to control the communications interface 1602 to transmit to and/or receive from other nodes requests, resources, information, data, signals, or similar.

Optionally, the base station 1600 may comprise a memory 1603. In some embodiments, the memory 1603 can be configured to store program code that can be executed by the processing circuitry 1601 to perform the methods described herein in relation to base stations. Alternatively or in addition, the memory 1603 can be configured to store any requests, resources, information, data, signals, or similar that are described herein. The processing circuitry 1601 may be configured to control the memory 1603 to store any requests, resources, information, data, signals, or similar that are described herein.

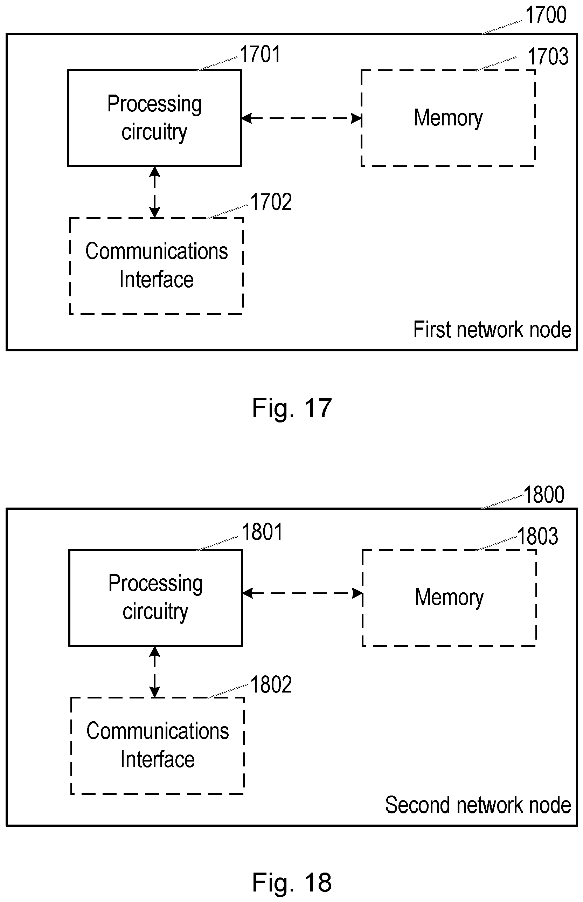

Fig. 17 is a block diagram of a first network node 1700 according to various embodiments. The first network node is not covered by the claims and is described for illustration purpose. The first network node 1700 comprises processing circuitry (or logic) 1701. The processing circuitry 1701 controls the operation of the first network node 1700 and can implement the methods described herein in relation to a first network node 1700. The processing circuitry 1701 can comprise one or more processors, processing units, multi-core processors or modules that are configured or programmed to control the first network node 1700 in the manner described herein. In particular implementations, the processing circuitry 1701 can comprise a plurality of software and/or hardware modules that are each configured to perform, or are for performing, individual or multiple steps of the methods described herein in relation to network nodes.

In some embodiments, the first network node 1700 may optionally comprise a communications interface 1702. The communications interface 1702 can be for use in communicating with other nodes, for example a wireless device, a base station or another network node.

For example, the communications interface 1702 can be configured to transmit to and/or receive from other nodes requests, resources, information, data, signals, or similar. The processing circuitry 1701 may be configured to control the communications interface 1702 to transmit to and/or receive from other nodes requests, resources, information, data, signals, or similar.

Optionally, the first network node 1700 may comprise a memory 1703. In some embodiments, the memory 1703 can be configured to store program code that can be executed by the processing circuitry 1701 to perform the methods described herein in relation to base stations. Alternatively or in addition, the memory 1703 can be configured to store any requests, resources, information, data, signals, or similar that are described herein. The processing circuitry 1701 may be configured to control the memory 1703 to store any requests, resources, information, data, signals, or similar that are described herein.

Fig. 18 is a block diagram of a second network node 1800 according to various embodiments. The first network node is not covered by the claims and is described here for illustration purpose. The second network node 1800 comprises processing circuitry (or logic) 1801. The processing circuitry 1801 controls the operation of the second network node 1800 and can implement the methods described herein in relation to a second network node 1800. The processing circuitry 1801 can comprise one or more processors, processing units, multi-core processors or modules that are configured or programmed to control the second network node 1800 in the manner described herein. In particular implementations, the processing circuitry 1801 can comprise a plurality of software and/or hardware modules that are each configured to perform, or are for performing, individual or multiple steps of the methods described herein in relation to network nodes.

In some embodiments, the second network node 1800 may optionally comprise a communications interface 1802. The communications interface 1802 can be for use in communicating with other nodes, for example a wireless device, a base station or another network node.

For example, the communications interface 1802 can be configured to transmit to and/or receive from other nodes requests, resources, information, data, signals, or similar. The processing circuitry 1801 may be configured to control the communications interface 1802 to transmit to and/or receive from other nodes requests, resources, information, data, signals, or similar.

Optionally, the second network node 1800 may comprise a memory 1803. In some embodiments, the memory 1803 can be configured to store program code that can be executed by the processing circuitry 1801 to perform the methods described herein in relation to base stations. Alternatively or in addition, the memory 1803 can be configured to store any requests, resources, information, data, signals, or similar that are described herein. The processing circuitry 1801 may be configured to control the memory 1803 to store any requests, resources, information, data, signals, or similar that are described herein.

Any appropriate steps, methods, features, functions, or benefits disclosed herein may be performed through one or more functional units or modules of one or more virtual apparatuses. Each virtual apparatus may comprise a number of these functional units. These functional units may be implemented via processing circuitry, which may include one or more microprocessor or microcontrollers, as well as other digital hardware, which may include digital signal processors (DSPs), special-purpose digital logic, and the like. The processing circuitry may be configured to execute program code stored in memory, which may include one or several types of memory such as read-only memory (ROM), random-access memory (RAM), cache memory, flash memory devices, optical storage devices, etc. Program code stored in memory includes program instructions for executing one or more telecommunications and/or data communications protocols as well as instructions for carrying out one or more of the techniques described herein. In some implementations, the processing circuitry may be used to cause the respective functional unit to perform corresponding functions according one or more embodiments of the present disclosure.

### ABBREVIATIONS

At least some of the following abbreviations may be used in this disclosure. If there is an inconsistency between abbreviations, preference should be given to how it is used above. If listed multiple times below, the first listing should be preferred over any subsequent listing(s).
- DC: Dual Connectivity
- EN-DC: E-UTRA NR Dual Connectivity
- LTE-DC: LTE Dual Connectivity
- MCG: Master Cell Group
- MN: Master Node
- MR-DC: Multi-Radio Dual Connectivity
- NR-DC: NR-NR Dual Connectivity
- NE-DC: NR-E-UTRA Dual Connectivity
- SCG: Secondary Cell Group
- SN: Secondary Node
- SRB: Signalling Radio Bearer
- 1x RTT: CDMA2000 1x Radio Transmission Technology
- 3GPP: 3rd Generation Partnership Project
- 5G: 5th Generation
- ABS: Almost Blank Subframe
- ARQ: Automatic Repeat Request
- AWGN: Additive White Gaussian Noise
- BCCH: Broadcast Control Channel
- BCH: Broadcast Channel
- CA: Carrier Aggregation
- CC: Carrier Component
- CCCH SDU: Common Control Channel SDU
- CDMA: Code Division Multiplexing Access
- CGI: Cell Global Identifier
- CIR: Channel Impulse Response
- CP: Cyclic Prefix
- CPICH: Common Pilot Channel
- CPICH Ec/No: CPICH Received energy per chip divided by the power density in the band
- CQI: Channel Quality information
- C-RNTI: Cell RNTI
- CSI: Channel State Information
- DCCH: Dedicated Control Channel
- DL: Downlink
- DM: Demodulation
- DMRS: Demodulation Reference Signal
- DRX: Discontinuous Reception
- DTX: Discontinuous Transmission
- DTCH: Dedicated Traffic Channel
- DUT: Device Under Test
- E-CID: Enhanced Cell-ID (positioning method)
- E-SMLC: Evolved-Serving Mobile Location Centre
- ECGI: Evolved CGI
- eNB: E-UTRAN NodeB
- ePDCCH: enhanced Physical Downlink Control Channel
- E-SMLC: evolved Serving Mobile Location Center
- E-UTRA: Evolved UTRA
- E-UTRAN: Evolved UTRAN
- FDD: Frequency Division Duplex
- FFS: For Further Study
- GERAN: GSM EDGE Radio Access Network
- gNB: Base station in NR
- GNSS: Global Navigation Satellite System
- GSM: Global System for Mobile communication
- HARQ: Hybrid Automatic Repeat Request
- HO: Handover
- HSPA: High Speed Packet Access
- HRPD: High Rate Packet Data
- LOS: Line of Sight
- LPP: LTE Positioning Protocol
- LTE: Long-Term Evolution
- MAC: Medium Access Control
- MBMS: Multimedia Broadcast Multicast Services
- MBSFN: Multimedia Broadcast multicast service Single Frequency Network
- MBSFN ABS: MBSFN Almost Blank Subframe
- MDT: Minimization of Drive Tests
- MIB: Master Information Block
- MME: Mobility Management Entity
- MSC: Mobile Switching Center
- NPDCCH: Narrowband Physical Downlink Control Channel
- NR: New Radio
- OCNG: OFDMA Channel Noise Generator
- OFDM: Orthogonal Frequency Division Multiplexing
- OFDMA: Orthogonal Frequency Division Multiple Access
- OSS: Operations Support System
- OTDOA: Observed Time Difference of Arrival
- O&M: Operation and Maintenance
- PBCH: Physical Broadcast Channel
- P-CCPCH: Primary Common Control Physical Channel
- PCell: Primary Cell
- PCFICH: Physical Control Format Indicator Channel
- PDCCH: Physical Downlink Control Channel
- PDP: Profile Delay Profile
- PDSCH: Physical Downlink Shared Channel
- PGW: Packet Gateway
- PHICH: Physical Hybrid-ARQ Indicator Channel
- PLMN: Public Land Mobile Network
- PMI: Precoder Matrix Indicator
- PRACH: Physical Random Access Channel
- PRS: Positioning Reference Signal
- PSS: Primary Synchronization Signal
- PUCCH: Physical Uplink Control Channel
- PUSCH: Physical Uplink Shared Channel
- RACH: Random Access Channel
- QAM: Quadrature Amplitude Modulation
- RAN: Radio Access Network
- RAT: Radio Access Technology
- RLM: Radio Link Management
- RNC: Radio Network Controller
- RNTI: Radio Network Temporary Identifier
- RRC: Radio Resource Control
- RRM: Radio Resource Management
- RS: Reference Signal
- RSCP: Received Signal Code Power
- RSRP: Reference Symbol Received Power OR Reference Signal Received Power
- RSRQ: Reference Signal Received Quality OR Reference Symbol Received Quality
- RSSI: Received Signal Strength Indicator
- RSTD: Reference Signal Time Difference
- SCH: Synchronization Channel
- SCell: Secondary Cell
- SDU: Service Data Unit
- SFN: System Frame Number
- SGW: Serving Gateway
- SI: System Information
- SIB: System Information Block
- SNR: Signal to Noise Ratio
- SON: Self Optimized Network
- SS: Synchronization Signal
- SSS: Secondary Synchronization Signal
- TDD: Time Division Duplex
- TDOA: Time Difference of Arrival
- TOA: Time of Arrival
- TSS: Tertiary Synchronization Signal
- TTI: Transmission Time Interval
- UE: User Equipment
- UL: Uplink
- UMTS: Universal Mobile Telecommunication System
- USIM: Universal Subscriber identity Module
- UTDOA: Uplink Time Difference of Arrival
- UTRA: Universal Terrestrial Radio Access
- UTRAN: Universal Terrestrial Radio Access Network
- WCDMA: Wide CDMA
- WLAN: Wide Local Area Network

## Claims

1. A method performed by a wireless device for handling group signalling of secondary cell, SCell, configuration information, the method comprising:
receiving (1010) configuration information for at least a first SCell, wherein the configuration information comprises values for one or more fields in a set of fields, wherein the received configuration information is group configuration information and wherein the group configuration information comprises values for the one or more fields that are applicable to a group of SCells including the first SCell, wherein the group configuration information further comprises a list of SCells in the group of SCells that the group configuration information applies to;
determining (1020) whether to configure the first SCell according to the received configuration
information; and
configuring (1030) the first SCell based on the result of the determining (1020).

2. The method of claim 1, further comprising the step of:
utilising radio resources from the configured first SCell.

3. The method of claim 1 or 2, wherein the configuration information is received in a radio resource control, RRC, message.

4. The method of any of claims 1-3, wherein the set of fields comprises a first field, wherein the first field is a field for which an absence of a value in the received configuration information is to cause a default action in the wireless device to release, discard, delete or stop using a previously-stored value for the first field.

5. The method of claim 4, wherein the received configuration information does not include a value for the first field.

6. The method of any of claims 1-5, wherein the step of determining (1020) whether to configure the first SCell according to the received configuration information comprises:
determining that the values for the one or more fields in the received configuration information are to be used for the first SCell in place of any previously-stored values for the one or more fields.

7. The method of claim 6 when dependent on claim 5, wherein the step of determining (1020) whether to configure the first SCell according to the received configuration information comprises either:
performing the default action for the first field and releasing, discarding, deleting or stopping using the previously-stored value for the first field; or
ignoring the absent value for the first field and maintaining the previously-stored value for the first field for the first SCell.

8. A method performed by a base station for handling group signalling of secondary cell, SCell, configuration information, the method comprising:
sending (1210) group configuration information for a group of SCells comprising a first SCell to a wireless device, wherein the group configuration information comprises values for one or more fields in a set of fields;
the method **characterized in that** the group configuration information further comprises a list of SCells in the group of SCells that the group configuration information applies to.

9. The method of claim 8, wherein the method further comprises:
determining whether the values for the one or more fields in the group configuration information are to replace previously-stored values for the one or more fields from individual configuration information.

10. The method of claim 8 or 9, wherein an indication that values for the one or more fields in the group configuration information are not to replace previously-stored values for the one or more fields from individual configuration information is provided by a negative indication, or by an absence of a positive indication.

11. The method of any of claim 8-10, wherein each of the fields in the set of fields is an optional field.

12. A computer program product comprising a computer readable medium having computer readable code embodied therein, the computer readable code being configured such that, on execution by a suitable computer or processor, the computer or processor is caused to perform the method of any of claims 1-11.

13. A wireless device (110) for handling group signalling of secondary cell, SCell, configuration information, the wireless device (110) configured to:
receive configuration information for at least a first SCell, wherein the configuration information comprises values for one or more fields in a set of fields, wherein the received configuration information is group configuration information and wherein the group configuration information comprises values for the one or more fields that are applicable to a group of SCells including the first SCell, wherein the group configuration information further comprises a list of SCells in the group of SCells that the group configuration information applies to;
determine whether to configure the first SCell according to the received configuration information; and
configure the first SCell based on the result of the determining.

14. A base station (520) for handling group signalling of secondary cell, SCell, configuration information, the base station (520) configured to:
send group configuration information for a group of SCells comprising a first SCell to a wireless device (110), wherein the group configuration information comprises values for one or more fields in a set of fields;
the base station **characterized in that** the group configuration information further comprises a list of SCells in the group of SCells that the group configuration information applies to.

## Patentansprüche

1. Verfahren, das von einer drahtlosen Vorrichtung durchgeführt wird, zur Handhabung von Gruppensignalisierung von Konfigurationsinformationen von Sekundärzellen, SCells, wobei das Verfahren umfasst:
Empfangen (1010) von Konfigurationsinformationen für mindestens eine erste SCell, wobei die Konfigurationsinformationen Werte für ein oder mehrere Felder in einem Satz von Feldern umfassen, wobei es sich bei den empfangenen Konfigurationsinformationen um Gruppenkonfigurationsinformationen handelt und wobei die Gruppenkonfigurationsinformationen Werte für das eine oder die mehreren Felder umfassen, die auf eine Gruppe von SCells, die die erste SCell umfasst, anwendbar sind, wobei die Gruppenkonfigurationsinformationen ferner eine Liste von SCells in der Gruppe von SCells umfassen, für die die Gruppenkonfigurationsinformationen gelten;
Bestimmen (1020), ob die erste SCell gemäß den empfangenen Konfigurationsinformationen konfiguriert werden soll; und
Konfigurieren (1030) der ersten SCell basierend auf dem Ergebnis des Bestimmens (1020).

2. Verfahren nach Anspruch 1, ferner umfassend den folgenden Schritt:
Verwenden von Funkressourcen von der konfigurierten ersten SCell.

3. Verfahren nach Anspruch 1 oder 2, wobei die Konfigurationsinformationen in einer Funkressourcensteuerungsnachricht, RRC-Nachricht, empfangen werden.

4. Verfahren nach einem der Ansprüche 1 bis 3, wobei der Satz von Feldern ein erstes Feld umfasst, wobei das erste Feld ein Feld ist, bei dem das Fehlen eines Wertes in den empfangenen Konfigurationsinformationen eine Standardaktion in der drahtlosen Vorrichtung auslösen soll, um einen zuvor gespeicherten Wert für das erste Feld freizugeben, zu verwerfen, zu löschen oder seine Verwendung zu beenden.

5. Verfahren nach Anspruch 4, wobei die empfangenen Konfigurationsinformationen keinen Wert für das erste Feld umfassen.

6. Verfahren nach einem der Ansprüche 1 bis 5, wobei der Schritt des Bestimmens (1020), ob die erste SCell gemäß den empfangenen Konfigurationsinformationen konfiguriert werden soll, umfasst:
Bestimmen, dass die Werte für das eine oder die mehreren Felder in den empfangenen Konfigurationsinformationen anstelle jeglicher vorher gespeicherter Werte für das eine oder die mehreren Felder für die erste SCell verwendet werden sollen.

7. Verfahren nach Anspruch 6, wenn abhängig von Anspruch 5, wobei der Schritt des Bestimmens (1020), ob die erste SCell gemäß den empfangenen Konfigurationsinformationen konfiguriert werden soll, entweder umfasst:
Durchführen der Standardaktion für das erste Feld und Freigeben, Verwerfen, Löschen oder Beenden der Verwendung des vorher gespeicherten Wertes für das erste Feld; oder
Ignorieren des fehlenden Wertes für das erste Feld und Beibehalten des vorher gespeicherten Wertes für das erste Feld für die erste SCell.

8. Verfahren, das von einer drahtlosen Basisstation durchgeführt wird, zur Handhabung von Gruppensignalisierung von Konfigurationsinformationen von Sekundärzellen, SCells, wobei das Verfahren umfasst:
Senden (1210) von Gruppenkonfigurationsinformationen für eine Gruppe von SCells, die eine erste SCell umfasst, an eine drahtlose Vorrichtung, wobei die Gruppenkonfigurationsinformationen Werte für ein oder mehrere Felder in einem Satz von Feldern umfassen;
wobei das Verfahren **dadurch gekennzeichnet ist, dass**
die Gruppenkonfigurationsinformationen ferner eine Liste von SCells in der Gruppe von SCells umfassen, für die die Gruppenkonfigurationsinformationen gelten.

9. Verfahren nach Anspruch 8, wobei das Verfahren ferner umfasst:
Bestimmen, ob die Werte für das eine oder die mehreren Felder in den Gruppenkonfigurationsinformationen vorher gespeicherte Werte für das eine oder die mehreren Felder aus individuellen Konfigurationsinformationen ersetzen sollen.

10. Verfahren nach Anspruch 8 oder 9, wobei eine Angabe, dass Werte für das eine oder die mehreren Felder in den Gruppenkonfigurationsinformationen vorher gespeicherte Werte für das eine oder die mehreren Felder aus individuellen Konfigurationsinformationen nicht ersetzen sollen, durch eine negative Angabe oder durch das Fehlen einer positiven Angabe bereitgestellt wird.

11. Verfahren nach einem der Ansprüche 8 bis 10, wobei jedes der Felder im Satz von Feldern ein optionales Feld ist.

12. Computerprogrammprodukt, umfassend ein computerlesbares Medium mit computerlesbarem Code, der darauf enthalten ist, wobei der computerlesbare Code derart konfiguriert ist, dass bei Ausführung durch einen geeigneten Computer oder Prozessor der Computer oder Prozessor zum Durchführen des Verfahrens nach einem der Ansprüche 1 bis 11 veranlasst wird.

13. Drahtlose Vorrichtung (110) zum Handhaben von Gruppensignalisierung von Konfigurationsinformationen von Sekundärzellen, SCells, wobei die drahtlose Vorrichtung (110) konfiguriert ist zum:
Empfangen von Konfigurationsinformationen für mindestens eine erste SCell, wobei die Konfigurationsinformationen Werte für ein oder mehrere Felder in einem Satz von Feldern umfassen, wobei es sich bei den empfangenen Konfigurationsinformationen um Gruppenkonfigurationsinformationen handelt und wobei die Gruppenkonfigurationsinformationen Werte für das eine oder die mehreren Felder umfassen, die auf eine Gruppe von SCells, die die erste SCell umfasst, anwendbar sind, wobei die Gruppenkonfigurationsinformationen ferner eine Liste von SCells in der Gruppe von SCells umfassen, für die die Gruppenkonfigurationsinformationen gelten;
Bestimmen, ob die erste SCell gemäß den empfangenen Konfigurationsinformationen konfiguriert werden soll; und
Konfigurieren der ersten SCell basierend auf dem Ergebnis des Bestimmens.

14. Basisstation (520) zum Handhaben von Gruppensignalisierung von Konfigurationsinformationen von Sekundärzellen, SCells, wobei die Basisstation (520) konfiguriert ist zum:
Senden von Gruppenkonfigurationsinformationen für eine Gruppe von SCells, die eine erste SCell umfasst, an eine drahtlose Vorrichtung (110), wobei die Gruppenkonfigurationsinformationen Werte für ein oder mehrere Felder in einem Satz von Feldern umfassen;
wobei die Basisstation **dadurch gekennzeichnet ist, dass**
die Gruppenkonfigurationsinformationen ferner eine Liste von SCells in der Gruppe von SCells umfassen, für die die Gruppenkonfigurationsinformationen gelten.

## Revendications

1. Procédé réalisé par un dispositif sans fil pour gérer une signalisation de groupe d'informations de configuration de cellule secondaire, SCell, le procédé comprenant :
la réception (1010) d'informations de configuration pour au moins une première SCell, dans lequel les informations de configuration comprennent des valeurs pour un ou plusieurs champs dans un ensemble de champs, dans lequel les informations de configuration reçues sont des informations de configuration de groupe et dans lequel les informations de configuration de groupe comprennent des valeurs pour les un ou plusieurs champs qui sont applicables à un groupe de SCells comprenant la première SCell, dans lequel les informations de configuration de groupe comprennent en outre une liste de SCells dans le groupe de SCells auxquelles les informations de configuration de groupe s'appliquent ;
la détermination (1020) s'il faut ou non configurer la première SCell en fonction des informations de configuration reçues ; et
la configuration (1030) de la première SCell sur la base du résultat de la détermination (1020).

2. Procédé selon la revendication 1, comprenant en outre l'étape suivante :
l'utilisation de ressources radio à partir de la première SCell configurée.

3. Procédé selon la revendication 1 ou 2, dans lequel les informations de configuration sont reçues dans un message de commande de ressources radio, RRC.

4. Procédé selon l'une quelconque des revendications 1 à 3, dans lequel l'ensemble de champs comprend un premier champ, dans lequel le premier champ est un champ pour lequel une absence d'une valeur dans les informations de configuration reçues est destinée à provoquer une action par défaut dans le dispositif sans fil pour une libération, un rejet, une suppression ou un arrêt de l'utilisation d'une valeur préalablement stockée pour le premier champ.

5. Procédé selon la revendication 4, dans lequel les informations de configuration reçues ne comprennent pas une valeur pour le premier champ.

6. Procédé selon l'une quelconque des revendications 1 à 5, dans lequel l'étape de détermination (1020) s'il faut ou non configurer la première SCell en fonction des informations de configuration reçues comprend :
la détermination que les valeurs pour les un ou plusieurs champs dans les informations de configuration reçues doivent être utilisées pour la première SCell à la place de toute valeur préalablement stockée pour les un ou plusieurs champs.

7. Procédé selon la revendication 6 dépendant de la revendication 5, dans lequel l'étape de détermination (1020) s'il faut ou non configurer la première SCell en fonction des informations de configuration reçues comprend :
la réalisation de l'action par défaut pour le premier champ et la libération, le rejet, la suppression ou l'arrêt de l'utilisation de la valeur préalablement stockée pour le premier champ ; ou
l'omission de la valeur absente pour le premier champ et le maintien de la valeur préalablement stockée pour le premier champ pour la première SCell.

8. Procédé réalisé par une station de base pour gérer une signalisation de groupe d'informations de configuration de cellule secondaire, SCell, le procédé comprenant :
l'envoi (1210) d'informations de configuration de groupe pour un groupe de SCell comprenant une première SCell à un dispositif sans fil, dans lequel les informations de configuration de groupe comprennent des valeurs pour un ou plusieurs champs dans un ensemble de champs ;
le procédé étant **caractérisé en ce que**
les informations de configuration de groupe comprennent en outre une liste de SCells dans le groupe de SCells auxquelles les informations de configuration de groupe s'appliquent.

9. Procédé selon la revendication 8, dans lequel le procédé comprend en outre :
la détermination si les valeurs pour les un ou plusieurs champs dans les informations de configuration de groupe doivent ou non remplacer des valeurs préalablement stockées pour les un ou plusieurs champs à partir des informations de configuration individuelles.

10. Procédé selon la revendication 8 ou 9, dans lequel une indication que des valeurs pour les un ou plusieurs champs dans les informations de configuration de groupe ne doivent pas remplacer des valeurs préalablement stockées pour les un ou plusieurs champs à partir des informations de configuration individuelles est fournie par une indication négative, ou par une absence d'une indication positive.

11. Procédé selon l'une quelconque des revendications 8 à 10, dans lequel chacun des champs dans l'ensemble de champs est un champ facultatif.

12. Produit programme informatique comprenant un support lisible par ordinateur dans lequel est mis en œuvre un code lisible par ordinateur, le code lisible par ordinateur étant configuré de sorte que, lorsqu'il est exécuté par un ordinateur ou un processeur approprié, l'ordinateur ou le processeur soit amené à réaliser le procédé selon l'une quelconque des revendications 1 à 11.

13. Dispositif sans fil (110) pour gérer une signalisation de groupe d'informations de configuration de cellule secondaire, SCell, le dispositif sans fil (110) étant configuré pour :
recevoir des informations de configuration pour au moins une première SCell, dans lequel les informations de configuration comprennent des valeurs pour un ou plusieurs champs dans un ensemble de champs, dans lequel les informations de configuration reçues sont des informations de configuration de groupe et dans lequel les informations de configuration de groupe comprennent des valeurs pour les un ou plusieurs champs qui sont applicables à un groupe de SCells comprenant la première SCell, dans lequel les informations de configuration de groupe comprennent en outre une liste de SCells dans le groupe de SCells auxquelles les informations de configuration de groupe s'appliquent ;
déterminer s'il faut ou non configurer la première SCell en fonction des informations de configuration reçues ; et
configurer la première SCell sur la base du résultat de la détermination.

14. Station de base (520) pour gérer une signalisation de groupe d'informations de configuration de cellule secondaire, SCell, la station de base (520) étant configurée pour :
envoyer des informations de configuration de groupe pour un groupe de SCell comprenant une première SCell à un dispositif sans fil (110), dans laquelle les informations de configuration de groupe comprennent des valeurs pour un ou plusieurs champs dans un ensemble de champs ;
la station de base étant **caractérisée en ce que**
les informations de configuration de groupe comprennent en outre une liste de SCells dans le groupe de SCells auxquelles les informations de configuration de groupe s'appliquent.
